# EUROPEAN PATENT SPECIFICATION

(11) **EP 0 428 604 B1**
(45) Date of publication and mention of the grant of the patent: **20.11.1996**
(21) Application number: 89909519.4
(22) Date of filing: 14.08.1989
(51) Int. Cl.: A61B 3/02

(54) **AUTOMATED OCULAR PERIMETRY, PARTICULARLY KINETIC PERIMETRY**
SELBSTTÄTIGER OKULARE PERIMETRIE, INSBESONDERE KINETSCHE PERIMETRIE
PERIMETRIE OCULAIRE AUTOMATIQUE, NOTAMMENT PERIMETRIE CINETIQUE

(30) Priority: 12.08.1988 US 231764
(43) Date of publication of application: 29.05.1991
(73) Proprietor: VISMED, INC., San Diego, CA 92121 (US)
(72) Inventor: RORABAUGH, Dale, A., Rancho Santa Fe, CA 92667 (US); DAVIS, Neil, M., Eugene, OR 97403 (US); MANSFIELD, George, A., Jr., San Diego, CA 92122 (US); BRANCACCIO, Vince, Eugene, OR 97403 (US)
(74) Representative: Strehl Schübel-Hopf Groening & Partner
(86) International application number: US8903495
(87) International publication number: WO9001290

(56) References cited:
- EP-A- 0 164 439
- EP-A- 0 164 981
- DE-A- 3 143 882
- US-A- 3 883 235
- US-A- 4 260 227
- US-A- 4 349 250
- US-A- 4 429 961
- US-A- 4 490 023

## Description

### BACKGROUND OF THE INVENTION

### 1. Field of the Invention

The present invention concerns measurement of the visual field of a patient's eye. The present invention concerns visual field testing wherein the patient's eye fixates on a moving point and the administration of test stimuli, and the recovery of patient response data, is automatic.

### 2. Background of the Invention

The present invention is concerned with a method of visual field testing, and with automated test instrumentation for performing the method. As background to the invention, the physiological basic of ocular perimetry, and the pathological conditions diagnosable by ocular perimetry, will be discussed in abbreviated form. Next, the prior art methods of surveying the visual field will be reviewed. Next, certain specific prior art instrumentation will be summarized. Finally, the problems with existing ocular perimetry measurement instrumentation, and the requirements for improved instrumentation, will be discussed.

### 2.1 The Physiological Basis of Ocular Perimetry

The optical system of the human eye projects a visual image onto the retina, just as the lens of a camera focuses an image on film. The retina transcribes the visual image into a neural code that is transmitted through nerve pathways to the visual cortex of the brain.

The human visual system is specialized into two broad divisions: (i) central or macular vision which is capable of resolving small detail and detecting color, and (ii) peripheral vision that can detect images in a panoramic as wide as 180° in the horizontal extent. An imaginary line of sight, the visual axis, connects the point of an eye's visual fixation on any given visual image to the fovea centralis. The fovea centralis is a small depression in the center of a retina area known as the macula lutea. The macula lutea is a highly specialized portion of the retina that can resolve details smaller than one minute of arc. The resolving capability of the macula outside of the fovea is less, and decreases with increasing distance from the fovea.

The receiving area for all macular fibers occupies about half of the total primary receiving area for visually afferent fibers. The remaining visual fibers projecting from areas of the retina that are peripheral to the macula are highly sensitive to light stimuli (when adapted to darkness) but their capacity for seeing fine details (visual acuity) is low.

This physiological structure of the human eye creates a vision field which is sometimes called an "island of vision", or a "hill of vision". The "island of vision" represents visual contrast sensitivity. It rises out of a surrounding "sea of blindness". The island shows a tall, narrow peak at its center. The peak, the point of visual fixation and highest visual acuity, represents the fovea, or retinal area of greatest resolution of visual stimuli. The size, or horizontal dimension, of the island is a measure of the extent of vision. The vertical height of the island is a measure of contrast sensitivity. The height of the island diminishes with distance from the peak (the point of visual fixation representing the fovea) just as the sensitivity of the extra-foveal retina diminishes. Beyond the edges of the island, or "shoreline", there is no sight because there are no rod or cone retinal receptors. No stimulus, no matter how strong, can be seen beyond the "shoreline" of the island of vision.

Pathologic conditions of the eye and/or brain, and the corresponding topography of the island of vision, may exhibit two types of defects: general and focal. A general defect in the island of vision is characterized by an overall diminution in visual sensitivity, or a reduced height profile of the island. This phenomenon occurs most commonly because the brightness or the clarity of an image is diminished at the retina. It may be due to (i) reduction of light entering the eye due to small pupillary opening; (ii) impaired media transparency due to corneal, lens or vitreous opacities; (iii) unfocused retinal image due to uncorrected refractive error, or (iv) reduced retinal sensitivity due to advanced age or high myopia.

Focal defects in the island of vision are characterized by deformities in its landscape. They are generally produced by disease in the neural portion of the visual pathway extending from the retina to the visual cortex.

There are two major types of focal defects: depressions and scotomas. A depression is an indentation of the surface of the island of vision. It shows up as a deformation of an isopter, or closed curve of equal contrast sensitivity derived as a horizontal slice through the island of vision.

On a horizontal slice, or isopter, through the island of vision a depression appears as an inward shift of a portion of that isopter - a warpage of the normal contour of that isopter. On a vertical slice through the island of vision the equivalent portion of the meridional profile will show a downward sagging.

A scotoma, or dark spot, is a pit, or well, in the island of vision. It is represented on a kinetic map as an area in which one or more targets are not perceived. It has a limiting border, which encircles and defines the defect on all sides. On static maps a scotoma appears as a trough in the profile.

The value of visual field testing of the eye is that it permits identification and localization of the responsible pathological condition. The localization of the pathological condition to a particular portion of the visual pathway is accomplished by a two step process: (i) identifying the relative attributes of the focal defects, and (ii) interpreting the meaning of these attributes in accordance with a knowledge of visual pathway anatomy.

A focal defect is defined by its location, shape, depth, and by the slope of its margins. The visual field of each eye is plotted on a chart that represents the visual space as the patient sees it. A commonly used combined chart shows the visual field seen by the patient's left eye upon the chart's left-hand side and the visual field seen by the patient's right eye upon the chart's right-hand side.

For each eye's chart section, perpendicular horizontal and vertical meridians that intersect at the central point of fixation conveniently divide the visual field of each eye into four quadrants. Coordinates in these quadrants are established both by radial meridians (defined by distance in degrees from the horizontal fixation meridian (0°-180°), and by circles of eccentricity (defined by the distance in angular degrees of their circumferences from the point of fixation).

Visual field testing plots the island of vision, including defects located therein, upon coordinate charts. When the plotted defects are studied as to location, shape, depth, and slope of margins, then the underlying pathological conditions giving rise to such defects may be diagnosed, and appropriate therapy applied.

### 2.2 Previous Methods of Surveying the Visual Field

Three methods are commonly used to survey the island of vision landscape, and to search for contour irregularities indicating visual defects. These visual test methods used to survey and map the visual landscape, or field of vision, are commonly called the (i) kinetic, (ii) static threshold, and (iii) static suprathreshold methods.

In kinetic perimetry a moving, kinetic, visual test target is advanced from a non-seen area outside the visual field radially into the visual field until it is first detected by the patient. The test target is of fixed stimulus characteristics, which stimulus characteristics represent a fixed height on the island of vision. Each time the test target is radially advanced toward the center of the visual field from a different direction a visual threshold point is mapped for the stimulus presented by that particular target. The joining of the visual threshold points so mapped forms a line of equal contrast sensitivity called an isopter.

Altering the brightness or size of the test target is equivalent to altering the altitude at which the island of vision is examined. For each different target brightness or size a different isopter will be mapped. In effect, the kinetic method of surveying the island of vision maps a series of horizontal slices through the island of vision. The outermost contour so mapped, beyond which even the brightest and largest test targets cannot be seen, represents the shoreline of the island of vision. Other isopters map the topological contours of the island of vision.

It will later be seen that at least two different adaptions of this previous kinetic perimetry method of surveying the island of vision are realized by the apparatus and method of the present invention. These adaptations will be called (i) reverse kinetic perimetry and (ii) modified reverse kinetic perimetry. They respectively represent the second and first preferred operational modes of the present invention.

A second previous method of surveying the island of vision landscape is the static threshold method. The static threshold method of surveying the island of vision probes various single points in the visual field with a static, or stationary, stimulus that is increased in brightness or size until the patient is first able to perceive it. The magnitude of the stimulus that evokes the first perception of the test target at any point defines the visual threshold at that exact point in the visual field. When this method is successively applied to a number of points, usually along a single meridian, then it creates a two-dimensional profile of the visual field which is equivalent to a vertical slice through the island of vision. This static threshold method is the most common previous method of automated testing of the visual field by computerized instruments.

A previous modification, being a simplification, of the previous static method of visual testing is the static suprathreshold method. In mapping visual fields by this previous method, the target's stimulus magnitude is set suprathreshold, or brighter than the normal expected threshold for each point upon the island of vision to be tested. If a suprathreshold level target is not seen, especially after repeated presentations, then the visual field at this point is presumed to be subnormal threshold, and abnormal. Static suprathreshold method testing is, therefore, not quantitative. It is primarily useful only to make a threshold determination that contrast sensitivity at a particular point in the visual field is either normal or abnormal.

It will later be seen that the static methods are not normally implemented by the visual field test apparatus of the present invention. However, it will later be seen that the apparatus in accordance with the present invention is fully capable of conducting complete, quantitative, static threshold perimetry and also static suprathreshold perimetry. Indeed, it is improved for conducting both. Particularly, in accordance with the present invention the method of conducting static perimetry (of either the static threshold or static suprathreshold types) will be modified. This (iii) modified static threshold and suprathreshold perimetry consists of the (static) presentation of stimuli at successive approximations, both sub- and supra-threshold, to the actual visual sensitivity at a point upon the island of vision. This conduct of modified static perimetry is not a preferred operational mode of the present invention.

As might be expected from an apparatus in accordance with the present invention that will be found to be sufficiently versatile so as to perform (i) reverse kinetic perimetry, (ii) modified reverse kinetic perimetry, and (iii) modified static threshold and suprathreshold perimetry, the apparatus will also be found to be capable of optionally performing, as still yet another method in accordance with the present invention. This method will be seen to employ some of the concepts of threshold/suprathreshold static perimetry in combination with (ii) modified reverse kinetic perimetry. This perimetry will be called (iv) modified reverse kinetic perimetry with successive approximations to points on the island of vision.

The pertinent point of all these methods (i) through (iv) is simply that the apparatus and methods of the present invention, while transcending conventional perimetry apparatus and methods, have a relationship to such prior apparatus and methods. The present invention thus requires an understanding of such prior apparatus and methods and their limitations.

### 2.3 General Previous Instrumentation for Optical Perimetry

Ocular perimetry testing is performed with test instrumentation. The earliest visual field test instrument is the tangent screen. Von Graefe first described the plotting of "indirect vision" on a blackboard in 1855. In 1889, Bjerrum popularized perimetry as part of the standard clinical examination using a two meter by two meter, flat plane, black velvet "tangent screen". The tangent screen has served as a standard backdrop for visual field examination ever since.

The tangent screen has been increasingly displaced since the middle of the twentieth century by the bowl perimeter introduced by Hans Goldmann. In bowl perimetry a patient views a target projected onto a hemispheric bowl while the perimetrist introduces stimuli manually and records visual thresholds. The advantage of the bowl perimeter is that it allows measurement of the full extent of the visual field under highly controlled and reproducible testing conditions. Available stimuli vary widely in size and intensity and can be quickly turned on and off. Background illumination is strictly calibrated. The featureless white interior of the bowl minimizes distractions. The fixation of the patient's eye may be monitored by telescopic view. The stimulus can be introduced more easily than with tangent screen examination, and the recording of points is more convenient.

Comparative disadvantages of the bowl apparatus, and advantages of the tangent screen apparatus, include that only one testing distance is available (approximately one-third meter) with the bowl apparatus. Further, the size of the central field measurable by a bowl apparatus may be less than the size obtained with the standard tangent screen.

Conversely, the previous tangent screen apparatus also exhibits disadvantages. However, there is nothing intrinsically inferior with the tangent screen apparatus if its disadvantages can be overcome. (Insofar as the perimeter in accordance with the present invention resembles a tangent screen, it will later be seen that these disadvantages are substantially overcome.) One disadvantage with the previous tangent screen apparatus has been that (i) the ambient lighting, (ii) the stimulus intensities, and (iii) the rate of target movement were not truly uniform from one examination to another or from one test room to another. The apparatus in accordance with the present invention will later be seen to be used in a room of controlled ambient light intensity level, and to support the proper setting of this ambient light intensity. Moreover, it will be seen that during administration of testing in accordance with the present invention both stimulus intensity and rate of target movement will be strictly controlled and repeatable.

It should be noted that previous tangent screen devices of the type that use projected stimuli have also been able to accurately and repeatably control the rate of target movement in order to cause the appearance and disappearance of these targets within the field of vision. However, projected stimuli tangent screen devices lack standardized background and stimulus illumination, and they do not examine the entire peripheral visual field. These limitations will later be seen to be overcome by the apparatus in accordance with the present invention.

Another problem with use of the previous tangent screen apparatus has been that the examiner remains in full view of the patient and serves as a distraction to the patient's required maintenance of focus fixation. It will later be seen that the distraction of examiner is eliminated in the apparatus and methods in accordance with the present invention. Moreover, the problem with patient focus fixation, and with the monitoring of patient focus fixation, will also be seen to be substantially eliminated in the apparatus and methods in accordance with the present invention.

### 2.4 General Performance of Previous Instrumentation for Optical Perimetry

Automated and semi-automated visual field testers have been used in clinical practice since approximately 1970. Stimuli in these devices are not introduced by hand, but are instead introduced by displaying charts marked with fluorescent spots, or by turning on lights, or by projecting white spots on a screen or bowl. In some cases a computer directs the program of stimulus presentation (the test regimen). The particular test regimen may be, in some cases, directed to the detailed analysis of a specific suspected pathological condition of the eye.

Commercially available "automated" visual field instruments fall into general categories. Certain non-computerized instruments, available at relatively lower prices of a few thousand dollars, enable the performance of the static suprathreshold and the static threshold methods in an approximate test time of four minutes per eye. One computer-assisted instrument only serves to guide a manual examination, via threshold kinetic and static suprathreshold stimuli, in a normal visual examination lasting 20-40 minutes per eye.

Next, fully computerized suprathreshold visual field instruments, available at the cost of many thousands of dollars, perform static suprathreshold visual field testing at an approximate elapsed time of seven minutes per eye.

Finally, computerized static threshold visual field instruments are by far the most expensive, having prices ranging to $25,000. These instruments allow the performance of static and kinetic threshold tests at a test time of approximately 40 minutes per eye.

Curiously, data regarding instrument cost versus the test time for visual field instruments generally indicates a positive correlation, with increasing instrument cost resulting in increasing test time. This is contrary to the desired relationship. Administrators of visual field testing would seemingly be willing to pay more for an instrument that conducts visual field testing more efficiently in a shorter time.

The obvious explanation for the present positive correlation of instrument cost versus test time is that the more expensive instruments, and longer test times, perform visual field testing in a more thorough and exact manner. This is despite the fact that an initial, threshold, screening of the normalcy and acuity of the visual field could in many cases be conducted relatively quickly if a visual field test instrument were to be sufficiently effective in supporting this simple threshold screening. A mere seventy (70) data points on the landscape of the island of vision suffice for thorough threshold screening for visual defects. This simple concept deserves to be well considered. To repeat, seventy (70) accurate data points suffice for testing the human visual field sufficiently so as to identify the existence or non-existence of pathological conditions.

Yet previous threshold testing of visual acuity requires (i) skilled personnel, (ii) expensive instrumentation and (iii) up to forty (40) minutes to make this identification of the existence or non-existence of pathological conditions. There is obviously a problem in the previous apparatus and methods in getting the data on the requisite seventy (70) points. This problem is fundamentally rooted in the previous test regimens (as discussed in previous section 2.3) and in the instruments supporting these regimens (as discussed in this section). The test regimen of the present invention will be seen to be much better optimized and efficient in surveying the visual field. It will be seen to allow automated derivation of eighty (80) points in under four (4) minutes by employing new test regimens administered by new test instrumentation.

Of course if a defect(s) is (are) preliminarily detected during threshold screening, then it (they) may need to be further localized, quantified, and mapped with great precision -- implying a continuing comprehensive and exacting testing at least in the local area(s) of the perceived defect(s). An apparatus supporting visual field testing would optimally be very quick and efficient in some operational mode for simple threshold screening for visual defects. Meanwhile the apparatus would also, when operated in another mode(s) which might differ in any of the type, duration, regimen, or number of data points in the test, be capable of the most detailed and exacting testing directed to analysis of a particular defect and associated pathological condition. The perimeter device in accordance with the present invention will be seen to so function.

### 2.5 Specific Previous Instrumentation for Optical Perimetry

Only one known prior art instrument provides computerized kinetic threshold perimetry that is computerized in data acquisition somewhat similarly to the scheme implemented by the present invention. This instrument is the Perimetron™ perimeter that was available from Coherent, Palo Alto, California, circa 1983, at a 1983 cost of approximately $48,900. It enabled the performance of kinetic threshold and static suprathreshold visual field testing at an approximate test time of forty (40) minutes per eye. The perimetron used a bowl perimeter. It essentially automated the application of stimulus from various positions within the bowl. Although automated, the Perimetron™ perimeter has not, as a bowl-type perimeter, performed the preferred perimetry methods of the present invention.

A successor machine to the Perimetron™ perimeter is the Humphrey Field Analyzer (HFA). This device initially performed static perimetry by steering a light beam (when off) within a bowl perimeter and then turning the light beam on when it is at predetermined fixed locations. An improvement to perform kinetic perimetry in the HFA is announced circa 1988.

Additional prior art perimeters are shown within various U.S. patents. U. S. Patent No. 4,063,807 to Gelius, et al., discloses a parametric eye examination device having light-emitting diodes (LEDs) which are controllable for brightness. This device uses a hemispherical inner surface holding the lights. The instrument has a stationary fixation point which the patient stares at. One of one hundred and twenty-seven (127) LEDs is randomly presented to the patient. The patient indicates whether or not he/she has seen the illuminated LED by depressing a button. The test may be repeated with lights at different levels of brightness.

U.S. Patent No. 4,421,393 to Cohen, et al., describes a visual field perimetry device where the patient is seated in front of a semi-circular light bar having a plurality of LEDs located around the inner circumference. Pairs of the LEDs are randomly energized. The patient manipulates a force-control stick that provides a nulling signal for illuminating various pairs of LEDs. The patient's head is secured in a helmet rigidly fixed to the back of the seat to prevent side-to-side head movement.

U.S. Patent No. 4,346,968 to Melin, et al., discloses a conventional visual field testing system having a center light at which the patent is instructed to look with one eye during the examination. Other lights or series of lights are illuminated during the test and the patient indicates which of the lights he sees.

U.S. Patent No. 4,059,348 to Jernigan discloses an instrument which includes a mirror that positions a target beam of light sequentially at selected locations on a board. In one embodiment, a Polaroid photo allows immediate inspection after a test is made. The patient's eye is to remain fixed on a stationary point. However, movement of the eye is monitored.

U.S. Patent No. 4,421,392 to Pitts Crick, et al., discloses a perimeter for testing visual field light sensitivity that presents a moving spot or set of fixed spots visually to the patient. The patient fixes on an aperture on a card. A number of patterns having different gradations of density are made to appear. Patterns having gradations of density are used as a means of assessing contrast sensitivity. Again, the patient has to have his eye fixed on a particular point of reference.

U.S. Patent No. 2,564,794 to Shekels describes a vision plotting device. In operation, the patient fixes on a target and indicates when he sees a beam of light which is intermittently projected through a number of apertures in a backing plate. A recording head positioned over a chart is actuated when the patient holds his finger on a key to print a spot where he sees a dot of light. Thus each time a dot of light appears on the screen which the patient sees, a corresponding dot is printed on the chart. If the patient does not observe a light dot on the screen at any particular point when a light dot is displayed, then there is no record the dot was seen.

U.S. Patent No. 4,558,933 to Murr discloses a visual field testing device having a concave screen and selectively illuminated LEDs.

U.S. Patent No. 4,392,725 to Sheingorn discloses a visual field testing device having sequentially illuminated LEDs and a memory unit for recording and displaying test results.

U.S. Patent No. 4,634,243 to Massof, et al., discloses a glaucoma detection device which has a random dot background field and a movable stimulus pattern. A patient views a video display corresponding to the entire human field of normal vision. A random dot pattern is generated on the display. A smaller fixed stimulus pattern is superimposed on the random pattern. The fixed pattern is them moved in the random pattern and the patient presses a response key to indicate whether or not he sees the fixed pattern.

U.S. Patent No. 4,490,023 to Ludwig discloses an arrangement for test mark positioning in spheric perimeters which permits kinetic and static perimetric operation over the entire visual field.

### 2.6 A Specific Previous Method of Perimetry

A previous method of visual field examination of relevance to the present invention is described by Bertil E. Danato in the paper "Oculokinetic perimetry: a simple visual field test for use in the community" appearing in the British Journal of Ophthalmology, Volume 69 (1985) pp. 927-31. The paper describes a method of visual field examination which enables an unsupervised person to carry out self-assessment using only a paper test chart, a record sheet, and a pencil. It is entitled 'oculokinetic perimetry' because it is the subject's eye that moves and not the test target. However, the subject's eye moves only because the subject is told to move his/her eye, essentially by looking at and reading aloud a series of numbers, typically from 1 to 100, that are printed in a particular pattern somewhat like a starburst. As the subject looks at, and reads, each number he/she is also supposed to himself/herself note the detection or disappearance of an unmoving central test stimulus, normally a simple dot, by recording a record sheet. The record sheet typically presents numbers in respective positions as are the numbers upon the oculokinetic visual field test chart. The subject is directed to indicate that a central stimuli is not seen while the subject is (ostensibly) looking at a number by crossing out the corresponding number on the record sheet.

This self-administered test is very, inexpensive. It is alleged to provide non-ophthalmic health care workers with a simple means of performing perimetry in the community, and to allow susceptible people to carry out self-assessment of the visual fields at home. The test is intended to facilitate the detection and management of glaucoma, especially in underdeveloped countries.

Unfortunately for the simplicity and low cost of oculokinetic perimetry, it is extremely difficult for even intelligent, motivated, diligent, and well-instructed people to perform correctly. It is exceedingly difficult for a person to consciously look only in one place -- the target number -- located amongst many equally visually detectable equally enticing like places -- the other target numbers --while supposedly determining as to whether or not still another place -- a none too distinctive constantly visible central dot stimuli -- is visibly detectable in the peripheral field of a one eye's vision. The instinct, and showing, of the paper -- that an alternative method of visual field examination wherein it is the eye, and not the test stimulus, that moves would be of good value -- is indisputably correct. The implementation solely by presentation of a test chart and delivery of directions for use has not, however, proven successful in realizing this potential value.

### 2.7 General Requirements for an Improved Optical Perimeter

An improved perimeter for measurement of the human visual field will preferably satisfy a number of requirements substantially unrelated to the test regimen administered. The perimeter instrument should be fully automated with respect to both the presentation of stimuli and the monitoring of patient response. The entire visual field testing should be performed without technician involvement, such as being automated by a microprocessor. The perimeter in accordance with the present invention will be seen to be fully automated.

If a tangent screen, as opposed to a perimeter bowl, is employed then certain deficiencies in the tangent screen should be addressed. With a tangent screen it is generally difficult to have the background illumination even and calibrated; the screen instead exhibiting "hot spots" and dark areas. Since perception of a stimulus depends on the difference between target and background illumination, an even and known illumination throughout the field is critical. This problem can be, however, obviously overcome if the background illumination is precisely maintained, and if the stimuli illumination is calibrated. The calibration of the stimuli should preferably be done automatically.

Next, a tangent screen generally tests only the central field of vision as opposed to supporting testing of the total field of vision (such as may be performed with a bowl perimeter). A few eye diseases impair vision only in the peripheral visual field. The evaluation of this field is correspondingly necessary. It is possible to test the entire limits of a patient's field of vision with a tangent screen, but (i) the screen must be appropriately large, (ii) and the presentations upon such screen must be appropriately extensive, and (iii) the fixation point must be moved from its normal dead-ahead position. What is possible is, in practice, extremely uncommon. Particularly, the fixation point is seldom displaced from its reference position during performance of kinetic perimetry upon a tangent screen. Use of a tangent screen to test the far limits of a patient's field of vision is usually entered into only after a closer-in visual field detect has been detected, and even then typically only of the far visual field in the direction, or quadrant(s), of such defect.

Finally, when a target moves away from the point of fixation on the flat plane of a tangent screen then it becomes further from the patient's eye. This causes it to subtend a smaller visual angle and appear to be less bright. This is to be contrasted with the spherical screen of a bowl perimeter, where the patient's eye is located at the center of the hemispheric bowl and where the distance to targets, and the visual angles subtended by these targets upon the hemisphere of the bowl, is always the same. It should be recalled that the shoreline of the island of vision is defined as that boundary beyond which no visual stimuli, no matter how bright, can be seen. Consequently, if the objective is only to identify the shoreline of the island of vision, then there is no limitation to the use of a bright source moving on a tangent screen in order to do so. Normally, however, the objective is broader: to map the island of vision.

To meet the broader objective of mapping the island of vision by kinetic perimetry performed upon a tangent screen the target must be controlled in size and/or intensity. But this is difficult. A target stimulus on a tangent screen is normally produced by an incandescent lamp. It is hard to controllably repeatably obtain a dynamic range of intensities which is sufficiently large. Consider the vastly different elevations upon the island of vision. The ratio elevations representing visual sensitivity, is on the order of 10,000:1. Then consider where the brightest and most intense target source must be shown. It must be shown at the periphery of the island. Next, consider where the periphery of the island of vision lies on a tangent screen when the eye is fixating straight ahead. The periphery is far off to one side, and/or far off in elevation, and/or far off in depression from the central axis. Finally, consider what happens when a projection light source aligned along a central axis (or substantially along a central axis) is projected so as to produce a target upon the tangent screen in a position far off the central axis. The light reflects from the screen at an oblique angle. Little light returns to the eye. Producing a sufficiently intense target at the periphery of a tangent screen, at least by light projection, is very difficult.

It is a further requirement of automatic test instrumentation for examination of the field of vision that test stimuli at various illumination intensities should be numerous, and should readily occupy all positions necessary in order to accurately profile the island of vision. Generally, a goodly number of targets are required for mapping the island of vision by the static threshold and the static suprathreshold methods. However, in the previous kinetic perimetry method the number of generated optical stimuli, and their respective discreet positions, were required to be, and generally were, even more numerous and dense. The spatial density of targets at appropriate distributions of intensities should particularly always be realizable at levels that exceed the requirements of any particular, directed, test strategies which concentrate examination within certain areas of the field of vision.

In accordance with a desired capability to present diverse stimuli at diverse locations, an automated perimetry instrument should be versatile. A variety of (presumably software) programs for directing the presentation of particular stimuli should be available. These programs should execute in a substantially automated manner so that the operator of the perimeter need not learn, and relearn, special techniques of perimetry.

An automated perimetry instrument would preferably store patient response data and enable its retrieval at a later date. The data should be able to be recorded in a permanent record, such as by printing, and should be easily interpretable in such record form. It would optimally be the case that the perimeter itself would aid in the interpretation and reduction of this data. Mainly, the ocular test strategies could have built-in methods for estimating threshold fluctuations and patient reliability. For example, the automated perimeter could have stored in its memory age-standardized data from a large number of normal subjects in order to facilitate comparison of test results. Programs could be available that would permit the statistical comparison of data from tests between different subjects, and at different times upon the same subject.

### 2.8 Two Specific Requirements for an Improved Optical Perimeter

The requirements for an ideal automated perimeter obviously present a spectrum between the more obtainable and the less obtainable, between the more expensive and the more economical. However, at least two crucial specific, requirements appear possible of being addressed in a substantially improved manner.

The first of these requirements is the accuracy, and continuity, of the patient's visual fixation throughout the occasionally lengthy visual examination. Accurate fixation is a fundamental requirement for accurate perimetry. Exactly how accurately it should be expected that the patient should fixate, and continually fixate, is uncertain because little research has been done about the effect on parametric accuracy of tiny shifts in fixation. However, it is well known that fixation is never absolutely steady, even for the most conscientious and well-trained subject, and that some tolerance for small drifts in patient fixation is necessary during measurement of the visual field. In the prior art visual field testing determinations and redeterminations were readily tolerated which deviated by 3° or 4° in the circles of eccentricity from one determination to the next.

The prior art approach to insuring the best possible continuing patient fixation has been to monitor such fixation by one of several methods. The simplest method, although not automatic, was to have the technician view the patient's eye through a telescope or a television monitor. Some instruments use a "blind spot" method to monitor fixation wherein a stimulus is presented in the blind spot of the field of vision at sporadic intervals and, if the patient sees this stimulus, then it is an indication of poor fixation. Of course, it should be remembered that the blind spot itself is approximately 7° by 9° in the visual field. Exotic eye positional monitoring systems, such as those used by pilots in aiming the armament of a fighter plane, are also possible. The present invention will be seen to present a scheme of assuring accurate fixation which substantially alleviates any necessity of monitoring the fixation of the patient's eye. Nonetheless fixation will be seen to be checked in the present invention by presentation of an occasional stimuli in the blind spot -- similarly to previous fixation monitoring methods.

Prior art, constant fixation, visual field test methods create a great problem by making a fixation requirement upon the test subject which is demanding under the best of circumstances, which is randomly violated without knowledge of the test administrator and sometimes even without knowledge of the subject, which is highly perturbing to test results, and which is a general source of inaccuracy in visual field determinations. As previously stated, this inaccuracy is on the order of 3° to 4° minimum. Few medical observers of radiograms, CAT scans, sonagrams and like medical records that are presented in graphic form would tolerate 3 or 4 degrees random deviations -- both within the overall image and at totally random portions thereof -- from one observation to the next observation.

Impreciseness in measurement of the visual field may occasionally mask early recognition of subtle ocular pathology conditions, especially at the periphery of the retina. It is highly desirable that, short of tapping into the magnetic emanations of the optic nerve (such as by superconducting quantum interference devices) in response to optical stimuli, that the patient should be administered a visual field testing regimen that (i) can readily and easily be followed, (ii) is not readily subverted by malingering or subterfuge, (iii) is quick and efficient and totally automated, and most importantly, (iv) delivers repeatable results of improved accuracy and consistency. The improved visual field test method and apparatus in accordance with the present invention will be seen to realize these desires.

A second requirement that is susceptible to being addressed with substantial improvement is the initial cost, and life cycle operational cost, of automated perimeter instruments. Some of the most expensive perimeters cost approximately $25,000 and use continuous skilled technician/perimetrist labor during test administration sequences lasting up to 40 minutes per 2 eyes. These costs require that each of 6 patients per day (a nearly continuous usage) must be charged $155 to $160 for the perimeter-owning organization to break even. This budget includes approximately one-third of the test fee amount to pay for the cost of the perimeter instrument and the remaining two-thirds to pay for upkeep, repairs, overhead and technician labor. If the perimeter instrument were to be of substantially lower initial cost and, even more preferably, the requirement for skilled technician labor were to be reduced, then the cost of performing visual field examinations could be considerably reduced.

A perimeter would preferably conduct threshold visual acuity screening very rapidly, and would preferably also conduct detailed surveys of the island of vision (or sections thereof) rapidly. These requirements are in tension with the situation in the prior art where accurate perimetry for either threshold testing or for detailed diagnosis of the visual field requires numerous data points that vary in both spatial displacement and in intensity (especially for kinetic perimetry). These numerous data points have required commensurately lengthy time durations to gather the patient's response to the stimuli at each data point.

Short of sensing electrical impulses on the optic nerve for a fully automated man to perimetry-test-machine interface, the patient cannot be expected to give a response to visual stimuli at rates much higher than once every two seconds. Even if the patient's response is by voice, and even if the response is information-rich by having the patient state numbers "zero", "one", "two", etc. instead of just "yes" or "no", the patient can only give so much information before he/she fatigues, loses interest, or fails to cooperate. The test regimen conducted by the perimeter has to be very well thought out in order to get as much information as is possible as quickly, and as effortlessly to the patient, as is possible. The preferred visual field test regimen in accordance with the present invention will be seen to give, in only four (4) plus eighty (80) quickly derived data points, an excellent quantification of the field of vision.

The rapidity with which the perimeter in accordance with the present invention will extract quality information from the patient is of such a different order from prior perimeters that modern developments in the area of audiological testing should be considered by analogy. The days are past when an audiologist presents a tone at a certain frequency and volume and asks "Can you hear it?". Modern audiological test machines monitor rapid patient pushbutton responses to complex tonal and volume patterns like "dah dit-dit-dit-dit... Dah Dit-Dit-Dit-Dit... DAH DIT-DIT-DIT-DIT... **DAH DIT-DIT-DIT-DIT... DAH DIT-DIT-DIT-DIT**", etc. Conceptually, the fundamental difference between modern and antiquated audiological testing is the informational transfer rate, or density, at which data is recorded to a machine system regarding the patient's physiological auditory system. The present invention will enhance the efficiency and economy of gathering information regarding the patient's more complex visual system by roughly an order of magnitude over previous approaches, typically making a comprehensive quantitative eye exam roughly as fast and as inexpensive as a hearing test.

Accordingly, new approaches to the methods of performing ocular perimetry, and new automatic perimeter instruments for performing such methods, are desired.

### SUMMARY OF THE INVENTION

### 1. Summary Statement of the Invention

The present invention contemplates moving the visual target to which the (moving) eye is fixated instead of moving the test stimulus. This confers the advantages of (i) permitting the simultaneous presentation of multiple test stimuli for much faster testing and much reduced patient fatigue, and, in the preferred embodiment, (ii) using a common, fully automated, mechanism both to perform the visual field testing and to record the results thereof.

### 2. Summary Outline of the Apparatus and Principal Methods of the Invention

Starting from the kinetic perimetry discussed above, in which the patient's eye is fixated upon a first reference light source and a second stimulus light source is moved within the patient's visual field, the present invention is embodied in the method for surveying the visual field of a patient's eye disclosed in attached claim 1 and in the perimeter apparatus disclosed in attached claim 6.

The perimeter apparatus of the invention presents visual stimuli on a plane similarly to a tangent screen, but is preferably enclosed in a housing like the enclosure of a bowl perimeter. Extensive test methods, and preferred test regimens in accordance with such methods, are entirely automated under control of a computer. Patient responses upon presentation of visual stimuli are preferably audible, and are spoken into and understood by the perimeter. A coordinate strength of the present invention concerns, however, the new methods of perimetry that are preferably performed by the new perimeter apparatus.

In accordance with attached claim 1, a first, base, method of the present invention is a reverse of kinetic perimetry. A visual stimulus, called the target stimulus, to which the (moving) eye is fixated is moved. The test visual stimulus (stimuli) -- regarding which the recognition(s) or non-recognition(s) by the patient is (are) indicative of visual sensitivity at tested points within the visual field -- are not moved. This will be recognized to be the reverse of kinetic perimetry wherein the target stimuli is fixed while the test stimuli are moved. This method is accordingly called reverse kinetic perimetry (RKP).

This RKP method is totally and generally efficacious for visual field examination. However, it is preferably used in relation to other methods of the invention only for the limited purpose of plotting the blind spot of the visual field. Still other, second and third, methods that are each still further modifications of reverse kinetic perimetry will preferably be used for actual testing of the visual field. If a defect causing a blind area within the visual field is uncovered during such testing then detailed delineation of that area may still preferably be done using reverse kinetic perimetry.

In accordance with attached claim 3, a second, mainstay, method of the present invention is called modified reverse kinetic perimetry (MRKP). MRKP differs from reverse kinetic perimetry (RKP) in that the test visual stimuli (which are positionally fixed) are not continuously illuminated, but are only momentarily illuminated at times. The patient's recognition or non-recognition of these illuminations, as indicated, by his/her responses, are correlated in the perimeter with the locations and intensities of the moving fixation target stimulus and fixed test stimulus(i) in order to test and to plot the visual field of a patient's eye.

In accordance with attached claim 4, a third method of the present invention, called modified reverse kinetic perimetry with successive approximations to points upon the island of vision (MRKP-SA), is actually preferably performed in time sequence after the first method but before the second method. This third method is preferably used for the limited purpose of precisely deriving just a few points, typically four, within the patient's eye's visual field and upon the island of vision. These few points permit recognition of what the patient's eye should always see if it were everywhere normal. The points are used to define a reference island of vision to the actual visual sensitivity of the eye.

This reference island of vision is intended to be sized and adopted by the extrapolation from the four points. It is usually of smaller size than the actual island of vision. It is thus referred to as a "shrunken" island of vision. It should be understood, however, that the "shrunken" island of vision is simply a customized reference island of vision for a particular subject. If the subject exhibits greater than normal visual sensitivity at the four points then his/her "shrunken" island of vision may actually be larger than the normal for the subject's age.

Subsequent MRKP testing with the second method, given knowledge of this "shrunken" island of vision proceeds,very rapidly. It typically derives the entire visual field of the eye in only eighty (80) test points that are typically derived in twenty plus (20+) test iterations that typically transpire in less than four (4) minutes.

All three methods, and still other methods of testing the visual field in accordance with the present invention, are individually fully capable of testing the entire visual field, and each method alone is generally of superior efficacy relative to previous methods for so doing. The preferred combination of the three new methods and a fully automated perimeter apparatus permits in aggregate, however, that precise, repeatable, reliable, low-cost testing of the visual field is routinely performable in an unprecedentedly short time, usually less than four (4) minutes per eye and potentially as fast as thirty (30) seconds for some simpler tests.

### 3. Perimeter Apparatus of the Invention

The preferred embodiment perimeter apparatus in accordance with the present invention has a picture-size flat rear-projection screen that is preferably mounted in a light-controlled enclosure.

The perimeter presents a first light source, normally a single light emitting diode ("LED") but sometimes a small cluster of LED's, which is (are) commanded by a computer to (jointly) positionally move on the rear-projection screen. The first light source(s) is (are) typically red in color. The movement is accomplished by means of x-y axis step motors that induce movement similar to that of computerized drafting plotter.

The perimeter further includes a plurality, typically sixteen (16), of positionally fixed "test" or "stimuli" second lights located behind the rear-projection screen. Each of the "test" lights is typically 580 nanometers yellow light in color. One or more, typically four (4) of the "test" lights are caused by the computer selectively momentarily illuminate when it (they) are at various locations relative to the "target" light. The illuminations may be momentary or prolonged in accordance with the particular test regimen that is being performed (discussed in the following sections). The light intensity of all illuminations, whether prolonged or momentary, is strictly controlled.

At the time(s) of its illumination(s) each of the positionally fixed "test" light sources occupies an individual location in distance and in angle relative to the then existing position of the moving "target" light source. The patient responds to each "test" light source for which he/she detects the illumination(s) by a spoken response or, alternatively, by actuation(s) of a multi-position or pushbutton switch. If more than one "test" light source is simultaneously illuminated (as is typical) then the patient verbally indicates the number of light sources that are seen by verbally speaking a number. If multiple "test" light sources are simultaneously momentarily illuminated, and if not all are seen by the patient, a more detailed search is later conducted to individually identify the individual "test" light sources that are, and that are not, seen at any particular position of the moving "target" light source.

The computer that is causing the moving of the first, "target" light source, and that is also controlling the illumination(s) of one(s) of the second, "test" light sources at various positions of the "target" light source, both receives and records the patient's response data to the visual test stimuli of the "test" light source(s). Because the computer knows the relative positions in angle and in distance between the moving "target", light source and each of the one or more "test" light sources, and because it knows the intensity of the "test" light sources, it is able to calculate the visual field of the eye of the patient. This calculated three dimensional visual field, typically represented by the threshold perimeter shoreline of vision and several isopters on the island of vision, is plotted in two dimensions by the computer using the same x-y axis plotter mechanism otherwise used to move the first, "target", light source. All testing and plotting transpires in a completely automated manner.

For this reason of using one mechanism both to move a "target" light and to plot the test results, and for smoothness and for flexibility in all paths that are traced, the movement of the "target" light and/or the plotting of results preferably transpire by electromechanical, and, not solely by electronic, means. It will of course be understood that the moving target light(s) could be implemented electronically, such as by a television display. In such case the "target" lights might also appear on the television, or might be positioned in front of the cathode ray tube. It is alternatively possible that the moving "target" light might be implemented as a matrix consisting of multiplicity of positionally fixed lights and that the moving may proceed stepwise by successive illuminations amongst the lights, normally stepwise from light to light. The moving "pursuit" light might even appear on television.

If the perimeter screen is not enclosed, which it normally and preferably is, then the perimeter senses and displays the level of ambient, room, illumination in order that both (i) the room illumination may be better adjusted, and (ii) the light intensity of the "test" light stimuli may be adjusted in consideration of the actual ambient illumination.

The perimeter also displays messages, and prompts, to guide (to the limited extent necessary) conduct of the testing and also to indicate the stepwise progress of testing.

### 4. Reverse Kinetic Perimetry First Method of the Invention

The preferred embodiment of a perimetry apparatus in accordance with the present invention selectively performs, under software control, a reverse of the kinetic threshold method of visual field examination. This method is called reverse kinetic perimetry (RKP).

This RKP first method of the invention is not the most prevalent operational method of the perimeter for assessing visual sensitivity in terms of either the amount of time typically spent on the method or the amount of data typically derived with the method. The reason that the RKP method is not exclusively, or more often, or longer used is not because the method suffers from any inadequacies in determining the visual field, especially by comparison to all previous methods. The RKP method is preferably used for only a limited, but important, purpose in the present invention because its derivatives are even more powerful. This limited purpose is the location of the centroid, and the determination of the area of the blind spot, of the eye. The RKP method is first discussed in this section, however, in order that further, even more sophisticated, methods of the invention presented in later sections 5. and 6. may be better understood.

It should be recalled that in standard kinetic perimetry, a test stimulus is moved into the visual field of a patient's eye that is fixating straight ahead on a target stimulus. Reverse kinetic perimetry (RKP) in accordance with the present invention proceeds by moving an illuminated first, "target", source of light in order that it may be substantially continuously followed by, and substantially continuously visually fixated to the fovea of, the patient's eye. Simultaneously to this tracking of the moving first light source by the patient's eye a second light, the "test" light stimulus is continuously illuminated. The second light assumes various distances of separation from, and angles relative to, the moving first source of light.

The patient is queried to indicate those periods that he/she does or does not visually detect the continuous illumination of the second light while visually fixating upon the moving first light. Each change (in either sense) between periods of detection and periods of non-detection constitutes a temporal juncture. The relative positions, in both distances of separation and relative angles, of the first and of the second sources of light are recorded upon at least some, and normally on all, of these temporal junctures. The aggregate recorded relative positions thereby indicate the visual field of the patient's eye.

It should be recognized that the RKP method accords an expanded angular range relative to normal, fixed central fixation, kinetic perimetry. Particularly, when the test light stimulus is initially outside the visual field when the target stimulus is at its initial, reference, position but later moves into the visual field during moving of the target stimulus then testing may transpire to at least 60° in the nasal direction despite the presence of the patient's nose, to at least 60° superiorly despite the presence of the patient's brow, and to at least 75° inferiorly despite the presence of the patient's cheek.

In the preferred testing in accordance with the invention, a test stimulus is illuminated within the blind spot of the eye's visual field. The target stimulus is progressively moved until the test stimulus becomes seen by the patient. This is repeated so that the test stimulus exits the blind spot in different directions, typically at the four points of the orthogonal axis. The typical four points identified by the RKP method at the periphery of the blind spot of the eye define both the centroid and area of the blind spot.

### 5. Modified Reverse Kinetic Perimetry Second Preferred Method of the Invention

A second preferred method of the present invention for visual sensitivity, or visual threshold, screening is a modification, adding further sophistication, to the reverse kinetic perimetry (RKP) method described in the previous section. This second preferred method is called modified reverse kinetic perimetry (MRKP). It is a predominant operational model of the perimeter, being used to test some eighty (80) points within the field of vision. It is not, however, that test method that is secondly executed in the preferred testing sequence executed by the perimeter. That method, a third preferred test method, is even more sophisticated than MRKP. The MRKP second preferred method is accordingly discussed in this section in order that the third preferred method discussed in the next section 6. may be better understood.

In MRKP the second source of light is not continuously illuminated for the prolonged time duration during which the field of vision makes excursion onto the position of the second light source (by act of the eye's fixation following the moving first light source). Instead, the second light source is only illuminated momentarily at times. At these times the second light source is at predetermined distances of separation from, and angles relative to, the first source of light. The visual detection or non-detection of the second light's momentary illuminations by the patient is recorded. The recorded detections and non-detections are correlated with the corresponding distances of separation and angles between the at least one second light and the first source of light in order to determine a threshold sensitivity of the patient's eye to the illuminations.

A major advantage gained in MRKP is that multiple second, test, light sources may be simultaneously presented. The information on the patient's recognition of these simultaneous presentations may be simultaneously retrieved, such as by the patient's speaking of a single number "one", "two", "three", etc. The informational density of testing, and of data retrieval, is thus magnified in MRKP over all previous methods of perimetry that essentially obtain data upon only one point in the field of vision at one time.

If the momentary illuminations of the second light source are all very bright then the MRKP method may be used to derive (upon the collection of sufficient sample points and the numbers typically so collected are so sufficient) the rough shoreline of the island of vision, the blind spot, and/or any areas of scotomas causing complete absence of vision. Accordingly, even without any great sophistication, and without any variation in the illumination intensity of the second light source (which can simply be turned on full bright), regions of blindness can be very, very, quickly derived with the MRKP method.

However, the preferred performance of MRKP in accordance with the present invention yields much more information than just the shoreline of the island of vision: essentially the preferred method yields the threshold contours of an entire "island of vision" below which contours no point(s) on the patient's actual island of vision fall. The actual heights upon a patient's actual island of vision (the detailed point by point visual sensitivity of the patient) are normally not determined. It is, however, verified that all points on the patient's actual island of vision are above some "shrunken" island of visual sensitivity. The "shrunken" island of vision is completely customized for the individual patient by application of the third preferred method as will be explained. It need not be a subnormal island of visual sensitivity, and can actually be larger than is typical for the patient's age. It is merely a reference island relative to which it is determined that the patient's eye exhibits no significant visual defects by testing all regions of the patient's visual field.

In accordance with the present invention, the manner by which this tight screening for visual defects is obtained is by performing MRKP in a more subtle manner than merely momentarily illuminating the second light source to a fixed, bright, level of intensity. Instead, the intensity of the second light source is set at a predetermined variable level of illumination intensity upon each of the times of its momentary illuminations. These predetermined variable levels make the second light source to be within the contour of the "shrunken" island of vision (at its then separation distance and angle from the eye's focus) and therefore visible if the eye's vision is as expected at that location in the visual field. Any failure to detect illuminations of the second light may indicate and/or resultant from depressions, or areas of reduced sensitivity, in the island of vision as well as from scotomas.

The MRKP method thus gives an excellent, and very quickly performed, validation, or threshold test, of visual acuity at the level of a "shrunken" island of vision within the boundaries of the actual island of vision. Every test point obtained is variable in two spatial coordinates and in intensity, and is correspondingly a point that is rich in information. When these information-rich points are obtained quickly, as is the case in the preferred exercise of the MRKP method, then the entire test of the visual field is greatly accelerated with minimal patient fatigue.

### 6. Modified Reverse Kinetic Perimetry With Successive Approximations to Points Upon the Island of Vision: A Third Preferred Method of the Invention

The modified reverse kinetic perimetry (MRKP) method described in the previous section 5. was preferably conducting using knowledge of the contours of a patient's "shrunken" island of visual acuity. The "shrunken" island test standard is not the same for all patients and for all test environments. It is best fully customized for each individual patient and for each individual test environment.

How is this done? How can knowledge of the appropriate size and extent of the "shrunken" island of vision be gained? In accordance with the present invention realization of a fully customized island of vision for each patient is accomplished by performing a third preferred test method called modified reverse kinetic perimetry with successive approximations to points upon the island of vision (MRKP-SA). This third preferred method is preferably performed second in sequence during preferred testing with and by the perimeter. It typically transpires for three (3) iterations at each of four (4) points in the patient's visual field. At the rapid conclusion of applying a modest twelve (12) test stimuli and retrieving the patient detection/non-detection results the patient's "shrunken" island of vision is calculated by the computer.

The third preferred MRKP-SA method commences with strict control of the ambient light intensity of the test environment. In the preferred perimeter enclosure light intensity is adjusted to 31.5 apostilbes (1 asb = (1/π)·10⁻⁴ cd/cm⁻²). Alternatively, if the perimeter is implemented as a wall-mounted flat screen then the ambient light intensity of the test environment, or room, is quantitatively sensed. A display is presented to aid the test administrator to make a coarse adjustment of ambient lighting such as by means of adjusting a rheostat controlling the intensity of a room light source, by selectively energizing a variable number of discrete room light sources, or by selectively moving or occluding various room light sources. Fine compensation for ambient light level is further realized by the perimeter in accordance with the present invention during its presentation of test stimuli. This will, in accordance with the MRKP-SA method of the invention, make the test stimuli appropriately slightly brighter in an overilluminated test environment and appropriately slightly dimmer in an underilluminated test environment.

The third preferred MRKP-SA method in accordance with the present invention continues by quantitatively measuring (typically) four (4) actual points on the actual island of vision of a particular patient. The points are typically orthogonally aligned, initially at a modest elevation on the anticipated island of vision. The exacting determination of the actual points on the real island of vision transpires by a process of testing somewhat akin to joining threshold perimetry with modified reverse kinetic perimetry (which is itself derived from reverse kinetic perimetry which is, of course, the reverse of kinetic perimetry). Specifically, a "test" light source is first illuminated at a brightness normally above that expected for the local elevation of the island of vision (i,e., suprathreshold) of the corresponding point of the visual field. Normally four points are done at this "overilluminated", or suprathreshold, light stimulus level. Then, contingent upon patient recognitions, the test light source is again illuminated, now at an illumination level below that expected for the elevation of the island of vision of each of the points. Successive illuminations successively approximate the actual intensity sensitivity of the point upon the island of vision. The successive illuminations bracket with ever increasing accuracy the true visual sensitivity of the patient at typically four points (only) on the patient's island of vision.

The four-point height of the individual patient's island of vision typically becomes sufficiently well known (based on patient responses) after three (3) only iterations of variably illuminating each point. A complete, standard contour, "shrunken" island of vision profile is custom calculated in the computer based on the individual patient's demonstrated visual sensitivity as represented by the four sample points.

Testing by the second preferred MRKP method then ensues based on this customized "shrunken" island of vision. This second preferred method testing, when conducted over the nominal eighty (80) points, completely validates that, whatsoever the absolute extent and height of the patient's actual island of vision, significant defects in the nature of scotomas and/or depressions do not exist in this actual island save such defects will be detected.

A sharp reader may wonder why a perimeter that is capable of target intensity control, as well as (indirectly) target positioning, does not simply administer automated standard threshold perimetry. It may be recalled that in standard threshold perimetry the point of fixation remains fixed while the test stimuli increase in intensity. Why incur the complexity of modified reverse kinetic perimetry with successive approximations (MRKP-SA)? One reason is that multiple test stimuli may be simultaneously presented. This is impossible with standard kinetic perimetry. Multiple simultaneous test stimuli shorten the time of testing and reduce patient fatigue.

Another reason involves a small segment of the population exhibiting a particular pathology giving rise to extinction phenomena (wherein the presentation of plural test stimuli is more detectable than the presentation of individual test stimuli). The multiple test stimuli presentations in accordance with the present invention are the only way of testing such population. The extinction phenomenon is a special sign of a parietal lobe lesion and can only be detected if both nasal and temporal fields are examined simultaneously. The MRKP and MRKP-SA methods in accordance with the present invention test both the nasal and temporal fields simultaneously by act of presenting multiple stimuli. Indeed, the testing of the present invention is, for the specific populations exhibiting this pathology, even more sensitive than the testing of the general population. Accordingly, the third preferred MRKP-SA method of the invention is both accurate and reliable across the full range of patient visual islands, and is superior in speed and accuracy to alternative, conventional, methods that the preferred embodiment of a perimeter in accordance with the invention could also perform.

The same sharp reader may next wonder why, if the third preferred MRKP-SA method is so good, reversion is made to the second preferred MRKP method for full field visual screening. One answer is that the second preferred MRKP method is faster, checking each point in the visual field only once instead of three times. Of course, the selection of the appropriate intensity for each point in the visual field for fast testing with the MRKP method is supported by the previous identification of the "shrunken" island of vision using the first-performed MRKP-SA method.

Another answer is that the third preferred MRKP-SA method can always be reentered. Indeed, it may optionally be so entered particularly for the performance of a quantitative test of a large number of points, typically eighty (80), in the visual field. Such an expanded, detail, quantitative testing of the field of vision and exact plotting of a patient's actual island of vision is typically entered if, and when, the more rapid MRKP testing detects a defect in the visual field.

The third preferred MRKP-SA method of visual sensitivity testing at or near the surface of a patient's actual island of vision is fully extendable to performing a detailed local examination of actual local contours (actual visual sensitivity) on the island of vision, and not merely to threshold screening. The third preferred MRKP-SA method will be realized to meld some of the advantages of threshold perimetry in accurately determining the height of points on the island of vision with the power of kinetic perimetry for determining the position of the contours of the island. The power of the third preferred MRKP-SA method should be compared to geophysical mapping wherein both elevation and distance are important to accurate and efficient topographical surveying.

The dynamic methods of the present invention locate, and repeatably relocate, the circles of eccentricity with better than the previously typically 3° to 4° of accuracy. The MRKP-SA method is very powerful for accurately deriving heights, or visual sensitivity, on the island of vision and of defects therein. The topological maps of visual defects obtainable with the preferred apparatus and methods of the present invention are of superior quality.

### 7. Still Further Perimetry Methods of the Invention

The preferred embodiment perimetry apparatus in accordance with the present invention is fully capable of performing, under software control, standard threshold perimetry.

It is also possible in accordance with the present invention to perform a modified, improved, method of threshold perimetry in evaluation of the entire visual field. It should be recalled that normal threshold perimetry involves (i) fixing a patient's eye on a first light source, while (ii) positioning a second light source to be at different times at various distances of separation and at various angles relative to the first light source, while (iii) recording instances of a patient's visual detection or non-detection of the second light source at the various times during the positioning. This is followed by (iv) plotting the instances of the detections or non-detections versus the distances of separation and the angles in order to derive by the plotting a graphical representation of some portion of the visual field of the patient's eye.

In accordance with the present invention a modified, improved, method of threshold perimetry is accomplished by automatedly positioning with a mechanism (the x-y plotter) that physically moves the first (target) light source relative to the second fixed (stimulus) light source(s) in order to realize the various distances and angles of separation between the two light sources. Moreover, automatedly plotting transpires with this same mechanism that is otherwise and at other times used for the automatedly positioning.

### 8. Summary Advantages of the Invention

The present invention is directed to realizing diverse benefits. Cost is low. The initial ownership cost of the perimeter apparatus is minimized primarily by utilizing computerized control. Economy is further obtained because a major section of the apparatus -- an x-y plotter -- is used for the dual purposes of both automated administration of visual field testing and generation of a hardcopy (graphical) representation of test results.

The life cycle cost of apparatus ownership is minimized principally because the perimeter apparatus of the present invention is fully automated and requires no intervention by a skilled perimetrist. Furthermore, the apparatus conducts testing very quickly. A preferred method for optical threshold screening performed by the apparatus reliably and accurately gathers from one to four, and typically two or three, data points about every two seconds. A thorough automated test of threshold visual sensitivity, including production of a graphical hardcopy output, is completed in less than four (4) minutes. Certain limited tests are capable of being conducted in less than thirty (30) seconds.

Accuracy is high. The perimeter apparatus of the present invention is versatile to efficiently and effectively conduct test regimens of diverse types, including wholly new types in accordance with the new methods of the present invention, at any degree of precision required or desired, including at very high precision.

Extraction of data from the test subject is at unprecedentedly high levels. Most points within the field of vision that are extracted by the preferred, interoperative, methods and apparatus of the invention are at or very near to visual threshold sensitivity, and are accordingly very useful and important points bearing much information. These information-rich points are obtained quickly and without the repetitions, retracings, and trial and error retestings that attend previous perimetry methods and equipments.

The preferred methods of visual surveying in accordance with the present invention are entirely automated and are therefore reliably, repeatably, consistently, thoroughly, and economically performed to generate good results. However, in order to get superior results in threshold testing of the visual field at a very high speed, something more than an automated presentation of quality test stimuli, and an automated recovery of patient data in response to such stimuli, is required. The visual field test regimen must grab, and hold the full interest, attention, cooperation and involvement of the patient for the entire duration of testing. This is accomplished in the present invention by inducing the patient's eye to fixate upon a moving light source. The preferred test method of the present invention induces the patient to become actively involved. The patient's interest is piqued and his/her performance is maximized. Meanwhile, any fatigue resultant from an overly long fixation of the patient's eye on an unmoving spot is avoided. By the time that any patient adaptation, malingering, inattentiveness, and/or boredom sets in, the test regimen of threshold visual sensitivity in accordance with the present invention has been completed. Difficulties with (i) the patient's eye becoming tired, (ii) the patient's loss of interest, and (iii) retinal adaption -- all such as regularly arise in the conventional prior art methods of optical perimetry including the kinetic method -- are substantially eliminated. An eye following a moving light source is believed to be more apt to stay fixated on that moving light source than is a eye required to fixate on a non-moving source. Additionally, the patient's interest and cooperation is more easily maintained when the examination method, which may last several minutes, is not as boring and monotonous as are the prior art examination methods.

Furthermore, in accordance with all methods of the present invention it is possible to test to the outer limits of the patient's visual field. This visual field normally (in youth) extends 60° superiorly, 75° inferiorly, 60° nasally, and 100° temporally. This visual field is testable in accordance with the present invention despite the presence of the patient's nose, brow and cheek. This expanded latitude of testing is not possible with conventional perimetry of either the kinetic or threshold types wherein the patient fixates straight ahead and where the nose, brow and cheek get in the way of testing parts of the retina. Expanded latitude of testing is enabled in accordance with the present invention because the patient can be forced to look oppositely to where the transient stimulus is applied. That is, the patient's eye is rotated within his/her eye socket relative to his/her brow, nose, and cheek. At certain times a target stimulus will be momentarily illuminated at nasal, superior and inferior points while the reference first light upon which the patient's eye is fixated is respectively at temporal, inferior and superior positions. The required size of the rear-projection screen for a given angle between target and stimulus is also reduced.

It should further be recognized that both preferred perimetry methods of the present invention each entail a reversal of the normal, prior art, kinetic visual field examination wherein a test stimulus is moved into the visual field of a patient's eye that is fixating straight ahead on a reference stimulus. Particularly, in accordance with the present invention the test stimulus is fixed while the reference target stimulus is moved so that it may be followed by the patient's moving eye. The reverse kinetic perimetry (RKP), modified reverse kinetic perimetry (MRKP), and modified reverse kinetic perimetry with successive approximations (MRKP-SA) methods in accordance with the present invention each give an independent check upon results obtained with the conventional method(s). Each method independently supports examination of the entire visual field despite the patient's facial anatomy.

It should further be recognized that still further major variations of ocular testing are possible based on the flexible, programmable, computer-based, perimeter apparatus in accordance with the present invention. Particularly, the perimeter apparatus of the present invention may be driven and employed so that illuminations of the second light source(s) is (are) not momentary, but so that such source(s) remains on while the field of vision of the eye, tracking the moving reference source, gradually overlaps the second source(s). Particularly, a second light test stimulus may be fixed at separate times at respective (i) nasal, (ii) superior, (iii) inferior points. The reference target that is fixated by the patient's eye is then respectively moved (i) from a temporal position whereat the nasal stimulus point is not within the patient's visual field in a nasal direction until the patient's visual field crosses into the nasal stimulus point, (ii) from an inferior position whereat the superior stimulus point is not within the patient's visual field due to the patient's brow in an inferior direction until the patient's visual field crosses into the superior stimulus point, and (iii) from a superior position whereat the inferior stimulus point is not within the patient's visual field due to the patient's cheek in a superior direction until the patients visual field crosses into the inferior stimulus point. By this procedure, the angular separations of the respective stimulus points and the reference stimulus at each of the respective crossings are known. The crossings of the brightest test stimuli represent the maximum extent of the patient's visual field respectively in the nasal, superior and interior directions. The visual field is determined to its maximum extent, useful in detecting pathological conditions at the periphery of the field, despite interference from the patient's brow, cheek, and/or nose.

### BRIEF DESCRIPTIONS OF THE DRAWINGS

These and other attributes and aspects of the present invention will become increasingly clear upon reference to the following drawings and accompanying specification wherein:
Figure 1 is a three-dimensional representation of the island, or hill, of vision representing the sight in the right human eye;
Figure 2 is a graphical representation of a normal circular static field of the retinal sensitivity of the human eye plotted in isopter contour lines;
Figure 3 is a diagrammatic representation showing the island, or hill, of vision in relation to the left eye;
Figure 4 is a pictorial diagram showing the surveying of a visual field of a patient's eye in accordance with the present invention using a first embodiment of an ocular perimetry instrument in accordance with the present invention;
Figure 5a is a perspective view showing the first embodiment of an ocular perimetry instrument in accordance with the present invention;
Figure 5b is a perspective view showing a second embodiment of an ocular perimetry instrument in accordance with the present invention;
Figure 6 is a cut-away perspective view showing the interior of the first or the second embodiment of an ocular perimetry instrument in accordance with the present invention;
Figure 7 is a schematic block diagram showing the computer, input/output circuits, and peripheral devices that are part of either embodiment of an ocular perimetry instrument in accordance with the present invention;
Figure 8 is a schematic diagram showing the microstep driver used for x axis and y axis step motor control in either embodiment of an ocular perimetry instrument in accordance with the present invention;
Figure 9 is a schematic diagram showing the photo detector circuit part of either embodiment of an ocular perimetry instrument in accordance with the present invention;
Figure 10 is a schematic diagram showing the drivers of the target and fixation light emitting diodes (LEDs) in either embodiment of the ocular perimetry instrument in accordance with the present invention;
Figure 11a is a first flowchart showing preferred software executed by the computer of the ocular perimetry instrument for its initialization, and for its training to recognize a voice;
Figure 11b is a second flowchart showing preferred software for performing a blind spot test phase 1 by the method of reverse kinetic perimetry (RKP), which software is executed by the computer of an ocular perimetry instrument in accordance with the present invention;
Figure 11c is a third flowchart showing preferred software for executing a blind spot test phase 2 again by the RKP method, which software executed by the computer of an ocular perimetry instrument in accordance with the present invention;
Figures 11d and 11e are fourth flowcharts showing preferred software for conducting modified reverse kinetic perimetry with successive approximations (MRKP-SA) for testing the field of vision test particularly at four points, the software being executed by the computer of an ocular perimetry instrument in accordance with the present invention;
Figures 11f and 11g are fifth flowcharts showing preferred software for performing an 80 point modified reverse kinetic perimetry (MRKP) screening test of the visual field, the software being executed by the computer of an ocular perimetry instrument in accordance with the present invention;
Figures 11h and 11i are sixth flowcharts showing preferred software for performing an 80 point modified reverse kinetic perimetry with successive approximations (MRKP-SA) quantitative test of the visual field, the software being executed by the computer of an ocular perimetry instrument in accordance with the present invention;
Figure 12 is a diagrammatic representation of the simplified, conceptual, spatial relationships between a moving fixation stimulus and a fixed target stimulus at four different times of the momentary illumination of the target stimulus;
Figure 13 is a diagrammatic representation of an actual serpentine path traced by a moving fixation stimulus showing the positions of the fixation stimulus in this path at four different times when a one of two target stimuli is illuminated, testing thereby four points that are successively approximated on the island of vision.
Figure 14 is a diagrammatic representation of the angles and separations of the sixteen fixed target stimuli that are normally momentarily illuminated relative to a moving fixation stimuli (not shown);
Figure 15 is a graph showing 80 points that are tested within the visual field of the left eye during testing in accordance with the present invention;
Figure 16a is a graphical plot showing isopters of the visual field of a right eye, which isopters are derived by testing in accordance with the present invention, which isopters indicate a pathological condition in the eye's visual field;
Figure 16b is a graphical plot of the blind of an actual patient's eye superimposed over a normal standard blind spot, the contours of the actual blind spot having been derived by RKP testing in accordance with Figure 11b; and
Figure 16c is a graphical plot of a computed cross-sectional profile through the island of vision along on East to West axis through the blind spot of that actual visual field graphed in Figure 16a superimposed upon a normal cross-section of an island of vision scaled to the same overall visual sensitivity, the actual cross-sectional profile showing a pathological condition in the eye's visual field. This actual profile having been derived by either MRKP testing in accordance with Figures 11f, 11g or, more exactingly, MRKP-SA testing in accordance with Figure 11h, 11i.

### DETAILED DESCRIPTION OF THE INVENTION

### 1. Surveying the Visual Field

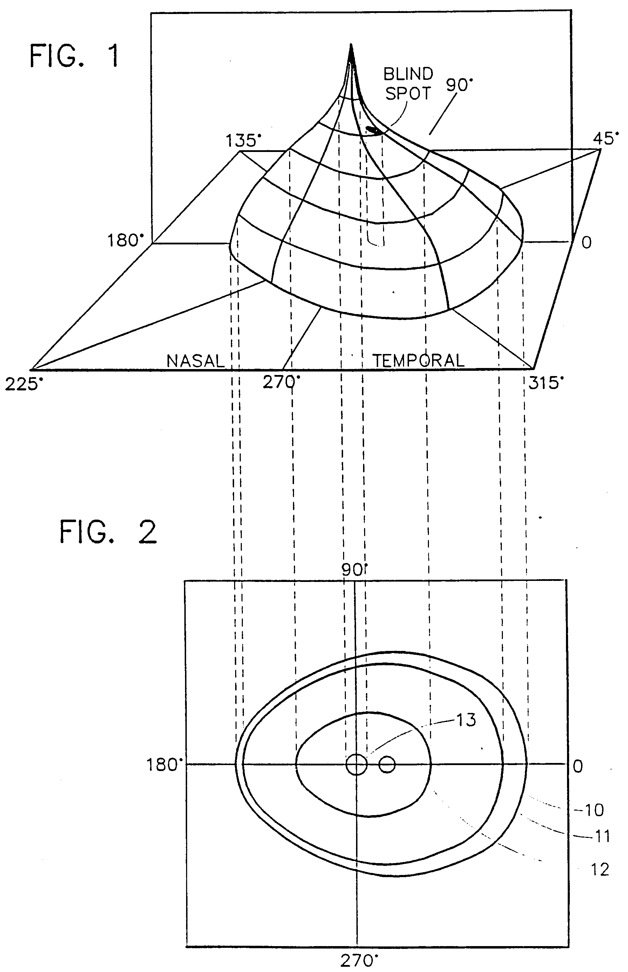

The present invention is directed to surveying the visual field of the human eye. The standard representation of the human visual field is shown in Figure 1. An island, or hill, of vision rises out of a flat plane, or sea, of blindness. Points on the island and sea are at various angles and distances from a central point that is the eye's fixation point. The plane, or sea, represents points to which the eye is insensitive. The island, or hill, exhibits a tall, narrow, peak at its center. This peak is the point of visual fixation, and highest acuity and visual sensitivity. This peak represents the fovea, or that retinal area of greatest resolving power and sensitivity to visual stimuli. The retinal sensitivity of this area is about 1 to .1 apostilbes. The shoreline of the island of vision has a retinal sensitivity of about 10⁴ apostilbes. The vertical, or height, scale is logarithmic. The dynamic range of contrast sensitivity of the eye is approximately 10⁴. This great range presents a challenge to accurate measurement of the visual field throughout the entire range.

The height of the island, or hill, represents the contrast sensitivity of the eye. The contrast sensitivity diminishes with distance from the peak, corresponding to diminishment in the sensitivity of the extra-foveal retina. The breadth of island is a measure of the extent of vision. Beyond the edges, or shoreline, of the island of vision there is no sight because there are no retinal receptors (rods or cones) within the eye.

The island of vision is not symmetric in slope or extent. Altitude, corresponding to contrast sensitivity, is lost rapidly on the nasal side. The landscape has a more general, prolonged slope on the temporal side. At a position about 15° temporal to fixation, a deep well with parallel vertical sides plunges from the island surface down to the plane, or sea, level of non-visibility. This well represents the physiologic blind spot, a "window" through which the retinal nerve fiber layer exits the eye at the optic nerve head. In this area, approximately 6° horizontally by 9° vertically in extent, there are no retinal receptors and no vision. Every point on the landscape on the island or of vision, or the visual field, corresponds to a point on the retina.

The visual field examination is equivalent to a search for contour irregularities in the island of vision landscape. Of the three major previous methods used to survey and map the visual field -- the kinetic, static threshold and static suprathreshold methods --the preferred methods of the present invention are most analogous to the kinetic method. A graphical representation of the results of a survey by the kinetic method is shown in Figure 2. The method of the present invention produces an identical representation (amongst other representations).

In the testing of visual fields by the previous kinetic method, a light of fixed stimulus characteristics, representing a constant altitude on the island of vision, is moved into the visual field until it is first detected by the patient. Because the test stimulus changes position, this stimulus presentation is called kinetic (moving). The stimulus is successively advanced toward the center of the visual field from a position of nonvisibility to a position of visibility, each advance being from a different direction relative to the center of the field. As the stimulus is advanced in each direction a visual threshold is mapped for a stimulus of that particular fixed characteristics (intensity and size) at the particular direction from which the target is advanced.

The joining of threshold points for a stimulus of a particular illumination level forms a line of equal contrast sensitivity called an isopter. The kinetic method is equivalent to mapping a series of horizontal slices through the island of vision. Four isopters 10-13, obtained by moving a corresponding four stimuli that are each of a different stimulus characteristic into the island of vision, are illustrated in Figure 2. The isopter 10 is the boundary, or shoreline, of the island of vision. It is the boundary beyond which no stimulus, no matter how great its luminance or size, may be detected. Efficient and effective optical threshold perimetry in determination of this threshold isopter 10 is one concern of the apparatus and methods in accordance with the present invention.

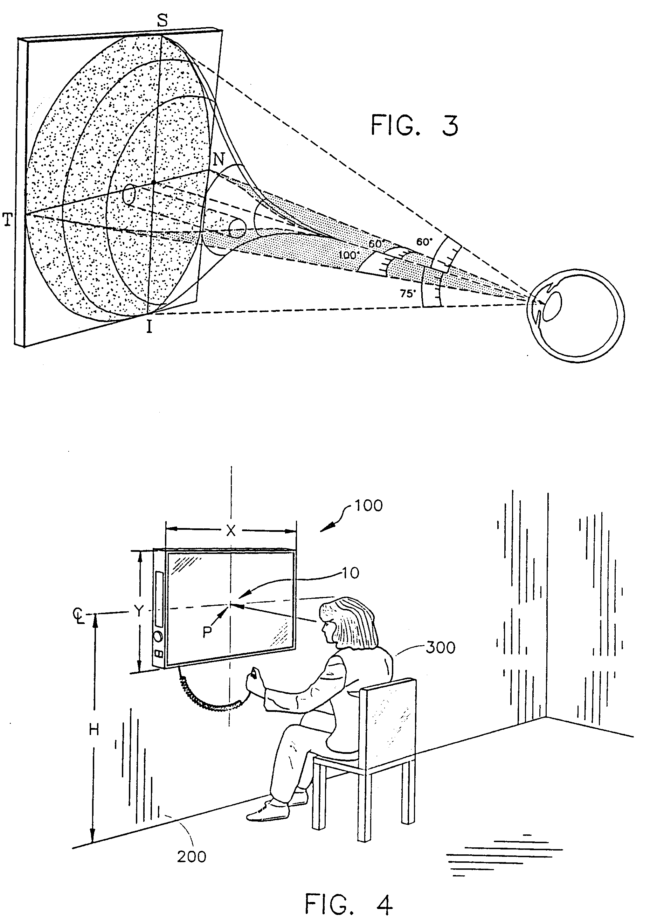

The present invention is, however, generally applicable to the surveying of the entire visual field in order to generate and present many isopters which will have identical appearance and significance to those shown in Figure 2. The extent of the island of vision is illustrated diagrammatically in Figure 3. The shoreline limits of the island, or boundaries of the visual field, typically extend from fixation 60′ superiorly (S), 60° nasally (N), 75° inferiorly (I), and 100° temporally (T) in youth. These limits are determined by the location and distribution of the retinal receptor cells. No adjustment of the head or eye position can extend the range of the visual field beyond these points. However, three of these innate limits of the visual field are not accessible by an eye which is fixating straight ahead. Particularly, the brow extends into the superior, the nose extends into the nasal, and the cheek extends into the inferior regions of the visual field. This may be easily verified for oneself by staring straight ahead and noting that one's own facial features appear at the far periphery of one's visual field in all directions save the temporal.

Previous methods of surveying the visual field, whether kinetic or static, require that the eye should be fixated straight ahead. This means that the ultimate limits of the actual visual field threshold may not be fully assessed in the superior, nasal, and inferior directions. This failure to examine the visual field at the full extent thereof is detrimental because certain deteriorations of the retina may start at the far peripheral boundaries thereof. Correspondingly, a thorough visual field examination should examine the absolute visual field thresholds of the eye even though it is not convenient to do so with previous test methodology and apparatus.

### 2. The Preferred Embodiment of an Ocular Perimetry Instrument in Accordance With the Invention

A diagrammatic representation of a first embodiment of an ocular perimetry instrument, or perimeter 100, is shown in its operational environment in Figure 4. The perimeter 100 is a flat field device of dimension X equaling approximately 32 inches and dimension Y equaling approximately 28 inches. It is mounted upon a wall 200 with its center at height H equaling approximately 48 inches. The perimeter 100 is approximately 3 inches in thickness. Its front is a planar rear-projection screen, normally a sheet of translucent plastic serving as a rear-projection screen for various light emitting diode (LED) light sources which are fixed to the rear side of the translucent front of the perimeter 100. The embodiment of the perimeter 100 shown in Figure 4 is normally viewed in a controlled illumination room by a patient 300 positioned in front of the screen at a distance D that normally equals approximately 14.2 inches. Other distances D could be defined in terms of the angles subtended threat. The patient is located with his/her eye to be tested roughly opposite the center of the screen.

Generally, the size of the perimeter 100 relative to the separation D from the patient's eye is not so great that the screen will extend from a central point P of fixation so far as an angle of 60° in the nasal direction, nor so far as 100° in the temporal direction (or a total of 160° horizontally). Neither will the perimeter 100 subtend an angle at the eye of patient 300 which extends either so far as 60° superiorly, or 75° inferiorly (or a total of 135° in the vertical direction). The screen 100 will, however, subtend angles at the eye of patient 300 at a distance D therefrom which angles exceed a total angular extent of 75° in the vertical direction and an angular extent of 100° in the temporal direction. In fact, the actual useful angles subtended by perimeter 100 will be approximately ±60° (120° total) in the vertical direction and nearly ±90° (180° total) in the horizontal direction at the eye of patient 300.

When, as will become evident during further discussion of the present invention, the patient's eye is allowed to fixate at the various margins (the top, bottom, left edge and right edge) of perimeter 100, then the angles subtended by the surface of perimeter 100 at the eye of patient 300 will be more than adequate to permit that introduction of illuminated objects at the opposite margins of the perimeter 100 will discern the absolute limits of the visual field of patient 300. The capability of the preferred embodiment of a perimeter 100 in accordance with the present invention to alter the patient's fixation point permits that the dimensions of a perimeter so performing should be smaller and/or the distance D should be greater than otherwise would be required. This capability saves on the size and expense of the perimeter, the required area of wall space required in the physician's office, and the required size of the room within which the visual field examination is conducted.

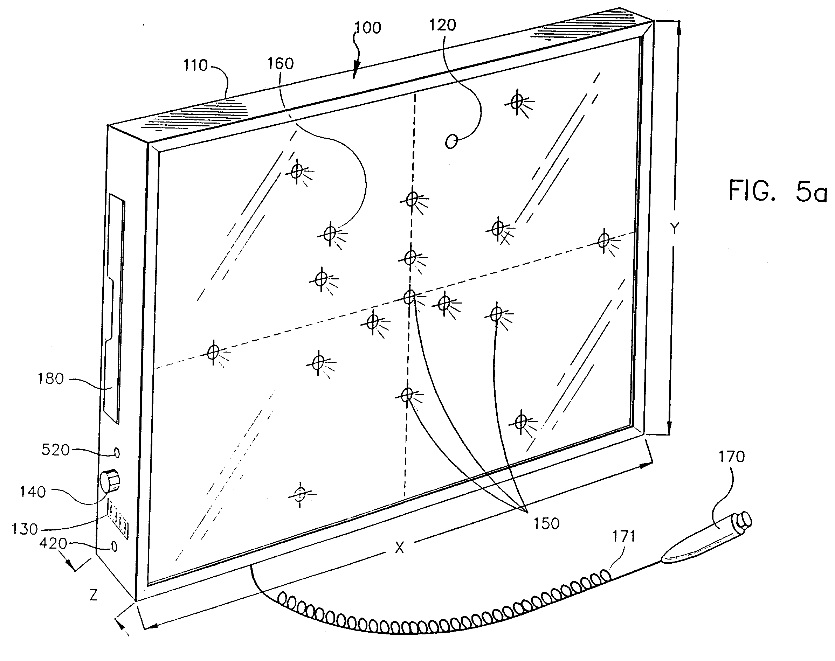

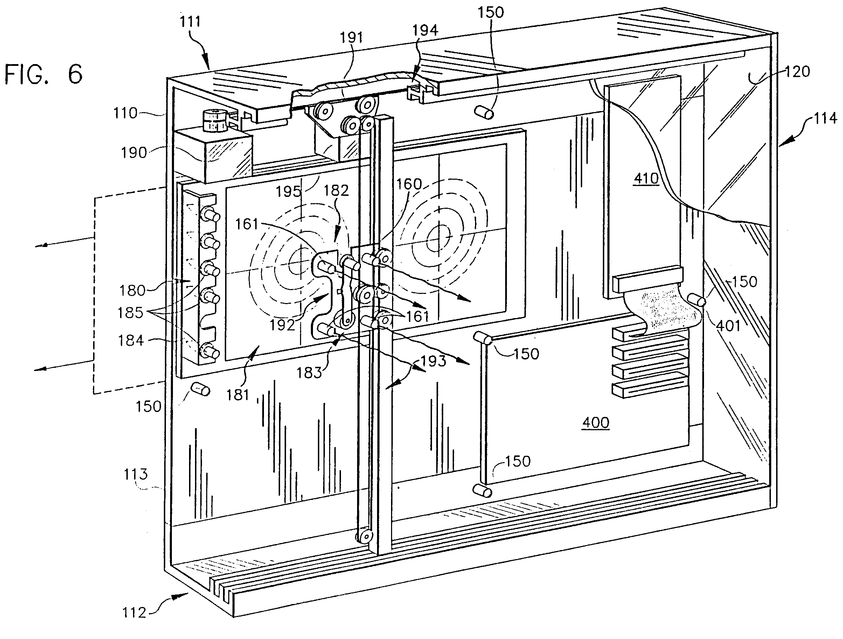

A detailed perspective view of that embodiment of a perimeter 100 in accordance with the present invention previously seen in Figure 4 is shown in Figure 5a. The perimeter is housed in a case 110 that mounts a rear projection screen 120 at its front surface. An alphanumeric display area 130 normally occupies a region at a one side, nominally the left side, of the rear projection screen 120. Appropriate operator messages involved with selection and administration of the test regimen are displayed in this area. For example, the display 130 variously displays (i) encoded-scale background illumination level, (ii) error conditions occurring during the conduct of testing, and (iii) the sequence of test events for observation by the test administrator (and possible response thereto, although such response is not normally required). The display of such messages is controlled by a computer (to be shown in Figure 6). The test selection and test control inputs by the operator in response to such messages are made through operator control 140, normally a simple knob controlling two single-pole single throw (SPST) switches.

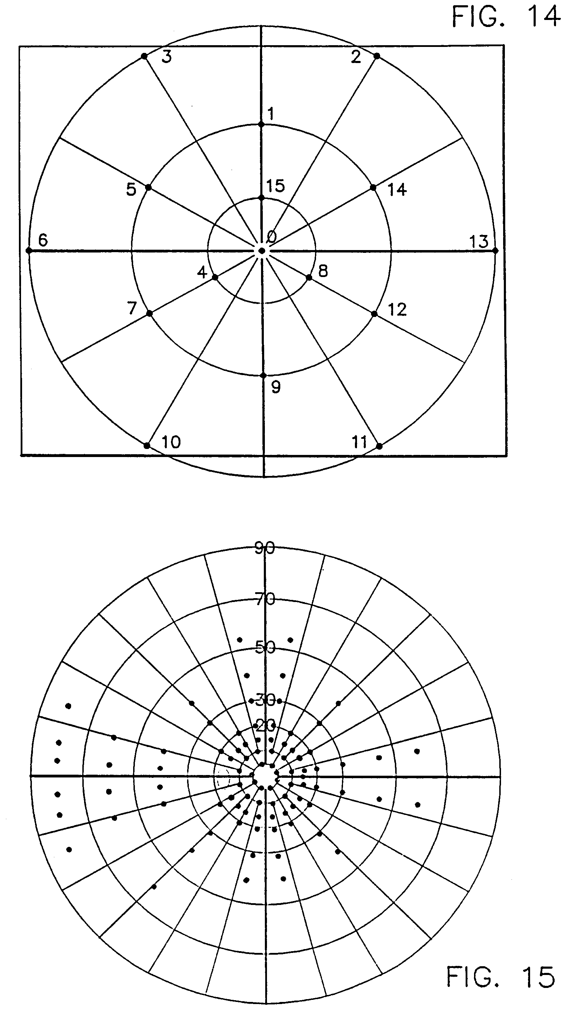

Also displayed on rear projection screen 120 at various times are one or more stimulus light sources. The stimulus light sources are preferably implemented by light emitting diodes (LEDs) 150 located at fixed locations. There are normally sixteen such LEDs located in the indicated pattern. This pattern, and the arbitrary numbering of the sixteen LEDs as numbers 0 through 15, is again shown in Figure 14.

A reference, fixation, light source 160, normally another single LED, but potentially a small cluster of LED's, is controllably moved to become positioned anywhere within the entire field of rear projection screen 120. The reference LED 160 is shown at an arbitrary position in Figure 5a. Not all of the fixed stimulus LEDs 150 and the moving fixation target reference LED 160 will be simultaneously illuminated.

A patient response push button 170 is connected to the perimeter 100 by cord 171. A microphone 520 mounted in case 110 permits reception of the patient's voiced responses. The push button 170, and the microphone 520, are the alternative means by which the patient 300 (shown in Figure 4) makes known his/her observations upon the momentary illuminations of the stimulus light sources 150.

A plotter paper tray 180 is capable of holding one or more sheets of plotter paper. The tray may be slid in and out from perimeter 100 in order that paper upon which visual field plots have been generated may be extracted for study and reference, and in order that additional unrecorded sheets of plotter paper may be installed as required.

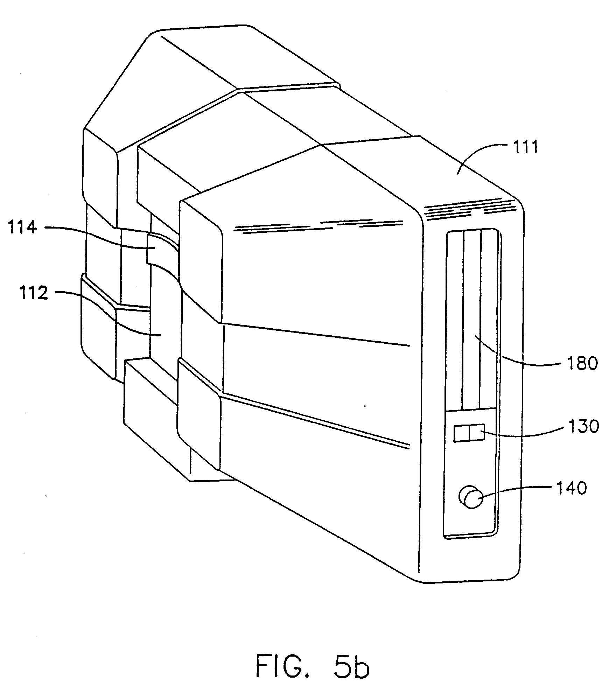

A second, preferred, embodiment of perimeter 100 is shown in Figure 5b. The case 110 of the embodiment shown in Figure 5a is simply expanded as case 111 in order to enclose the area between the screen 120 (not shown in Figure 5b for being inside case 111) and the opening 112 through which the patient observes the screen 120. The enclosing case 111 permits precise control of the ambient illumination level during visual field testing, and the precise positioning and positional maintenance of the patient's head on forehead rest 114.

The size of aperture 112, the considerable volume of case 111 which is approximately 32" width x 28" height x 18" depth, and the low ambient illumination within case 111, permit in combination that almost all patients can use the perimeter 100 without experiencing claustrophobia or being unduly distracted by the physical environment of the perimeter and the circumstances of its use. Indeed, the perimeter 100 functions in certain test methods (to be explained) entirely differently than a bowl perimeter which, in the encased embodiment of Figure 5b, it somewhat resembles. With a bowl perimeter the patient must be laboriously positioned and instructed.

The perimeter 100 requires neither extreme precision in the placement of the patient's head (not shown) at opening 112 nor in the instruction of the patient. The perimeter 100 may actually commence preliminary modified reverse kinetic perimetry with successive approximations (MRKP-SA) testing as sort of an "attract" mode similar to an arcade video game. The test cycles until it gets valid data. A patient may actually be enticed to look through aperture 112, to naturally start tracking the moving fixation light source 160 (shown in Figure 5a), and to either press the pushbutton 170 (shown in Figure 5a) or announce a number "one" through "four" (as instructed) with negligible, or even no, prompting or instruction.

The perimeter ergometrics and test regimens in accordance with the present invention should be appreciated during the ensuing discussion to be sufficiently well integrated and automated so that there is a finite chance that a patient receiving cursory instruction and placed alone in a room with the perimeter 100 will successfully administer himself/herself a comprehensive test of visual sensitivity. Complex testing of a complex physiological function with this degree of "slickness" is unprecedented, and makes the present invention an exemplary model of patient-friendly physiological test apparatus and test regimen design.

A cut-away perspective view of the preferred embodiment of a perimeter 100 in accordance with the present invention is shown in Figure 6. The case 110 includes an upper extrusion 111, and a lower extrusion 112. Both extrusions are of custom contour and are normally extruded from aluminum. The required contours of such extrusions permit, in addition to the necessary fitting of the back panel 113 and the rear projection screen 120, that the extrusions may mount (i) the x-axis rail 194 (which carries the x-axis carriage 191), and (ii) the other end of the y-axis rail 193 to that end which is borne by the x-axis carriage 191. This is simply to say that the case 110, and the extrusions 111 and 112 thereof, support, without modification, a conventional mechanical x-y plotter mechanism for free movement within the confines of the case. The case 110 also fits right and left end caps 114, which may be removed for maintenance.

The stimulus light sources, or LEDs, 150 may alternatively be fixed in position to case 110 instead of to the back side of screen 120. If the target light sources 150 are so alternatively affixed, then a typical five of the normal sixteen of these LEDs 150 area located as shown in Figure 6. Regardless of whether the LEDs 150 are affixed to case 110 or, as is normal, to the back of screen 120, the reference light source, or LED, 160, moves relative to each of case 110, screen 120, and fixed LEDs 150.

Normally only a single LED 160 is used as the fixation target. However, in the eye of some individuals there exists a blind spot substantially at the fixation point, meaning at the fovea, of the field of vision. In order for affected eye of such an individual to fixate upon any reference point, either moving or stationary, such reference "point" must be spatially extended. Accordingly, three additional LEDs 161, along with LED 160, are all illuminated as a moving reference, spatially extended, target light source for those certain individuals whose eye has a blind spot at or near the fovea that precludes the fixating of a single point light source. The individual having such macular degeneration is instructed to look toward the black center of target light sources 160, 161 and to follow their collective movement with his/her eye so that all four sources remain visible.

The reference target LED 160 is affixed to a y-axis carriage 192. The y-axis carriage 192 is controllably moveable in y-axis position upon y-axis rail 193 by y-motor 195 acting via a cable and capstan drive system. The y-motor 195, the y-axis rail 193, and the y-axis carriage 192 are entirely borne upon x-axis carriage 191. This x-axis carriage 191 is moveable in x-axis position along x-axis rail 194 under control of x-motor 190 in similar fashion to that of the y drive system. The normal and conventional carriage mechanism of an x-y plotter will be recognized.

The moveable y-axis carriage 192 also mounts, oppositely to reference LED 160, a releasably held marker pen 182. This pen may be selectively lowered by action of pen lift solenoid 183 into contact with graph, or chart, paper 181 which is carried upon the sliding paper plotter tray 180. Additional pens 185 of different colors may be picked up from pen reservoir 184 and individually selectively substituted for pen 182. At some time after completion of the examination of the visual field, and after computation in computer 400 of the parameters of the visual field resultant from such examination, the calculated parameters of the visual field are automatically plotted onto graph, or chart paper, 181 (normally 11" by 17"). The plotting is in a conventional form that is interpretable by an ophthalmologist. The plotting is preferably done in multiple colors for ease of interpretation.

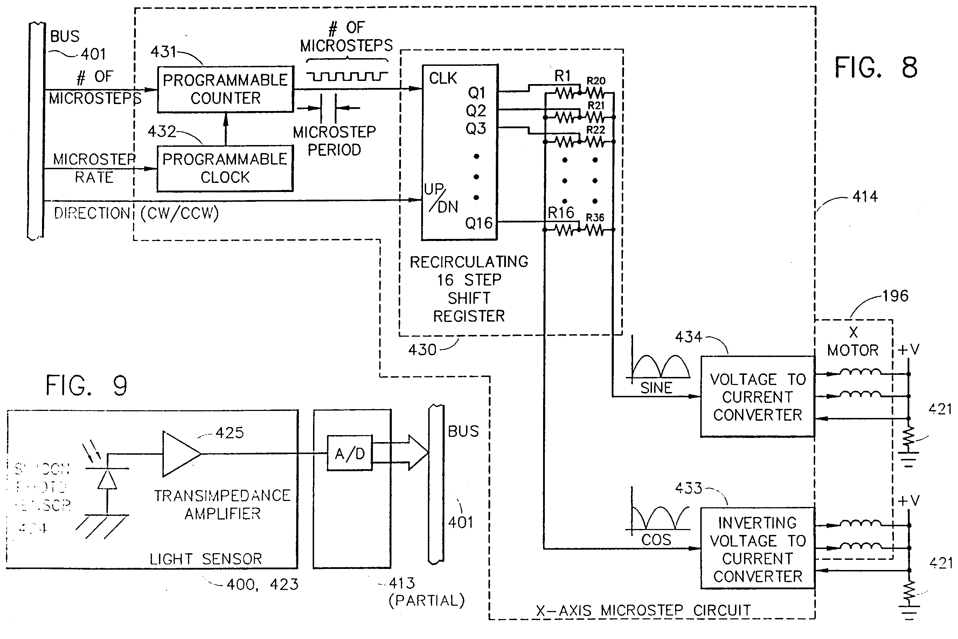

The exercise of the preferred embodiment of a perimeter 100 in order to perform visual field testing in accordance with the present invention is by computer 400 operating under programmed control. The computer 400 is normally the motherboard of a personal computer, normally of the XT type available from IBM Corporation and other suppliers. At one of the expansion slots normally available on the motherboard of computer 400 a custom electronics assembly, or I/O board, 410 is connected by ribbon cable 401. The communication to and from computer 400, and the control program resident therein, transpires through this I/O board 400. The functions implemented on the I/O board 400 are substantially conventional, and could be substantially derived in standard communications boards available from diverse manufacturers, with the exception of the microstepper function to be shown in Figure 8. The functions are preferably implemented in a customized I/O board 410 in order to save space, weight, and cost. The I/O board 410 will be understood to exhibit wired electrical interconnections (not shown) directed to both receiving information from (e.g., from the push button switch 170), or to directing control to (e.g., to the x-motor 190 and the y-motor 195), other elements within the perimeter 100.

### 3. Electrical Construction Details of the Ocular Perimetry Instrument in Accordance with the Invention

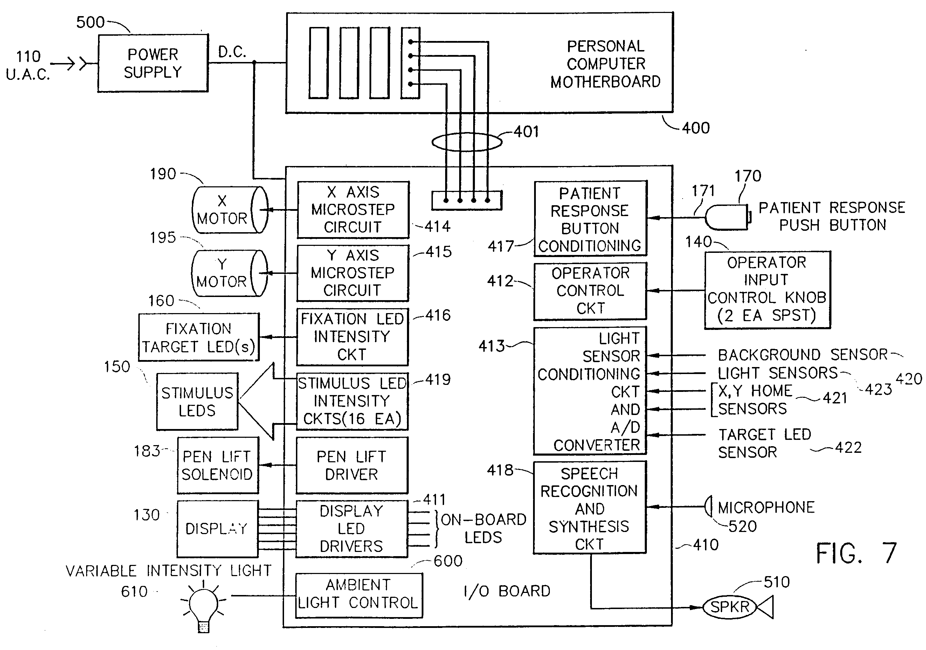

An electrical schematic block diagram of the control section of either embodiment of an optical perimetry instrument, or perimeter 100 in accordance with the present invention is shown in Figure 7. Instrument control is implemented by a PERSONAL COMPUTER MOTHERBOARD 400, including a software program which is normally fixed in unerasable memory such as PROMS or otherwise (not shown). Both the PERSONAL COMPUTER MOTHERBOARD 400 and the I/O BOARD 410 are supplied with direct current, D/C, power from POWER SUPPLY 500. The POWER SUPPLY 500 receives normal 110 V.A.C. power via a plug. Power on/off switches (not shown) for the POWER SUPPLY 500 and for the perimeter 100 are employed for power control. The PERSONAL COMPUTER MOTHERBOARD 400 communicates with the I/O BOARD 410 via ribbon cable 401 (previously seen in Figure 6) for receiving operator initiation and control of testing, for controlling in an automated manner the testing operations of perimeter 100, and for receiving the patient's responses to the test sequence.

In the idle condition, ready for control inputs, the program residing in the memory of, and running within, the PERSONAL COMPUTER MOTHERBOARD 400 will cause the DISPLAY LED DRIVERS 411 within the I/O BOARD 410 to display messages within the area of DISPLAY 130 (previously seen in Figure 5). These lighted messages within the area of DISPLAY 130 will solicit that the operator should initiate a test sequence by selecting one or more quantities with OPERATOR INPUT CONTROL KNOB 140. This knob preferably controls two ganged single pole single throw (SPST) switches. The switch closings are detected at OPERATOR CONTROL CIRCUIT 412 on the I/O BOARD 410. They are appropriately debounced and level transformed in order that they may be read through the data bus of PERSONAL COMPUTER MOTHERBOARD 400. Responsive to selections by the OPERATOR INPUT CONTROL KNOB 140, the program within the PERSONAL COMPUTER MOTHER BOARD 400 will cause the appropriate changing of DISPLAY 130 by selectively enabling the DISPLAY LED DRIVERS 411, which are also upon the data bus of the computer.

At such time as automated testing of the visual field by the perimeter 100 has been selected, the PERSONAL COMPUTER MOTHERBOARD 400 will interrogate via the LIGHT SENSOR CONDITIONING CIRCUIT AND A/D CONVERTER 413 (i) the background light intensity via BACKGROUND SENSOR 420, (ii) the position of the x/y carriage via X, Y HOME SENSORS 421, and (iii) the on condition of the illuminated FIXATION TARGET LED(s) 160 via the TARGET LED SENSOR ON 422. In accordance with the sensed conditions, the computer will adjust the intensity of all light sources and position the carriage of the x/y plotter mechanism.

If the perimeter 100 is enclosed within the case 110 shown in Figures 4 and 5a then the computer will indicate the background light illumination condition in display 130. The test administrator should adjust ambient illumination in accordance with the display 130, which is continuous and continuously updated by the PERSONAL COMPUTER MOTHERBOARD 400 in accordance with sensed background light intensity. At such time as the test administrator has adjusted ambient light to be approximately 31.5 apostilbes another selection with OPERATOR INPUT CONTROL KNOB 140 will cause PERSONAL COMPUTER MOTHERBOARD 400 to commence to conducting the selected automated test procedure.

If the perimeter 100 is alternatively cased in the preferred embodiment of case 111 shown in Figure 5b the PERSONAL COMPUTER MOTHERBOARD 400 will act through AMBIENT LIGHT CONTROL 600, essentially a digital-to-analog converter with an amplified power output signal, to control VARIABLE INTENSITY LIGHT 610. The VARIABLE INTENSITY LIGHT 610, and the BACKGROUND SENSOR 420 that responds to such LIGHT 610, are both within the interior of case 111 (shown in Figure 5b). The internal illumination level within case 111 is automatically set to approximately 31.5 apostilbes.

During the conduct of testing the PERSONAL COMPUTER MOTHERBOARD 400 will cause the X-AXIS MICROSTEP CIRCUIT 414 and the Y-AXIS MICRO-STEP CIRCUIT 415 to respectively drive the X-MOTOR 190 and the Y-MOTOR 195 so as to move the FIXATION TARGET LED(s) 160, upon which the patient focuses, within the field of the rear projection screen 120 (shown in Figure 5a). The FIXATION TARGET LED(s) includes both LED 160 and LEDs 161 previously seen in Figure 6. The intensity of the FIXATION TARGET LED 160 will be adjusted under computer control through the FIXATION LED INTENSITY CIRCUIT 416. If there were to be any change in the background light (which is detected by BACKGROUND LIGHT SENSOR 420 which is continuously interrogated through LIGHT SENSOR CONDITIONING CIRCUIT AND A/D CONVERTER 413), and/or the test regimen(s) was (were) to require a variation in the ambient light intensity or any selective illumination of the FIXATION TARGET LED(s) 160, then intensity variation may be accomplished under programmed control (within case 111).

The programmed control of PERSONAL COMPUTER MOTHERBOARD 400 also acts (i) through the FIXATION LED INTENSITY CKT 416 to vary the intensity of FIXATION TARGET LED(s) 160, and (ii) through the STIMULUS LED INTENSITY CKTS (16 EA) 419 to selectively vary the intensity (including "on" and "off") of individual STIMULUS LEDS 150.

During the course of testing the PERSONAL COMPUTER MOTHERBOARD 400 causes the STIMULUS LED INTENSITY CIRCUITS (16 EA) 419 to selectively illuminate ones of the STIMULUS LEDS 150. At the times of such selective illuminations the computer has knowledge of the position of FIXATION TARGET LED(s) 160, and the intensity of this (these) LED(s), by action of the control which it has previously effected through X-AXIS MICROSTEP CIRCUIT 414, Y-AXIS MICROSTEP CIRCUIT 415, and FIXATION LED INTENSITY CIRCUIT 416. At the times of these selective illuminations, the computer accumulates data regarding the patient response(s) either as (i) registered by the PATIENT RESPONSE PUSH BUTTON 170 and as communicated through cable 171 (shown in Figure 5) to PATIENT RESPONSE BUTTON CONDITIONING CIRCUIT 417, or else preferably as (ii) registered by spoken voice input to MICROPHONE 500 that is processed in SPEECH RECOGNITION AND SYNTHESIS CKT 418. If no patient response is received after an appropriate time at a particular position of the FIXATION TARGET LED(s) 160 and upon associated illuminations of ones of the STIMULUS LEDS 150, then the computer will assume that the patient has not seen any of the illuminations and the data recorded will so indicate.

It should be recognized that the binary signal produced by a PATIENT RESPONSE PUSH BUTTON 170 is not the only type of patient data input that might be made by mechanical, non voice, means to the perimeter 100 in accordance with the present invention. Particularly, the patient could be given a multiple position switch. The patient would indicate by positioning of a rotary knob or the like whether he/she has recognized nothing (the normal, home, default position), or has variously seen one, two, or three, or even four light sources upon a single illumination event. Multiple position switches are, however, believed to be (i) cumbersome, (ii) difficult for the patient to understand and learn, (iii) distracting to the patient who may attempt to look at the switch instead of the presented test stimuli, and (iv) difficult to reliably position in the dark.

Consequently, enhanced and sophisticated means for recovering data from the patient, and providing directions to the patient both before and during the test, are provided in accordance with the preferred embodiment of the perimeter 100 in accordance with the present invention. This sophisticated approach is based on the SPEECH RECOGNITION AND SYNTHESIS CIRCUIT 418. This speech recognition circuit, currently available as plug-in printed circuit board for a personal computers, permits a limited number of words such as "1", "2", ... "4" that are typically spoken by a variety of patients to be reliably recognized. These words are received in MICROPHONE 520 and decoded into digital representations recognizable by the computer.

The computer may optionally direct the provisioning of oral messages to the patient through the same SPEECH RECOGNITION AND SYNTHESIS CIRCUIT 418, this time driving the SPEAKER 510. The incorporation of the speech recognition and/or synthesis capability is not incompatible with the simultaneous and parallel incorporation of the PATIENT RESPONSE PUSH BUTTON 170. Rather, oral input and/or output is realized within and by the perimeter 100 as features that are well accepted by, and efficiently operable with, most non-mute hearing patients. The speech recognition and/or speech synthesis features are particularly beneficial in perimeters that are in intensive use for conducting visual field examination on a great number of patients, such as inductees into the armed forces.

After the completion of the administration of a test sequence, the computer is capable of, under program control, filtering the test data and repeating any portions of the test sequence for which results appear inconsistent or irrational. The computer subsequently displays (as will be next discussed) the data obtained both during initial test, and during any retests. The data displays indicate to a trained ophthalmologist observer whether malingering, subterfuge, or a true pathological condition has been uncovered during the testing. In certain more sophisticated, optional, configurations the computer is capable of accepting further audio inputs from the test subject and/or the test administrator indicating "go back" or "go slower" or "go faster". The computer responds to these inputs by adjusting the sequencing, resequencing, and/or rapidity of testing within predetermined parameters.

A detailed schematic diagram of one of the microstep circuits, for example X-AXIS MICROSTEP CIRCUIT 414 (previously seen in Figure 7) is shown in Figure 8. The PERSONAL COMPUTER MOTHERBOARD 400 (shown in Figure 7) sets, via its data bus which is carried on interconnection cable 401, the DIRECTION (CW/CCW) (either clockwise or counterclockwise) that a RECIRCULATING 16 STEP SHIFT REGISTER 430 will count. The computer also sets via the bus a # OF MICROSTEPS into PROGRAMMABLE COUNTER 431, and also a MICROSTEP RATE into PROGRAMMABLE CLOCK 432. The PROGRAMMABLE CLOCK 432 will produce pulses at a programmable rate into the PROGRAMMABLE COUNTER 431. The PROGRAMMABLE COUNTER 431 in turn passes a predetermined # OF MICROSTEPS at a MICROSTEP PERIOD representing that rate to the RECIRCULATING 16 STEP SHIFT REGISTER 430. The sixteen data output bits Q1-Q16 of the RECIRCULATING 16 STEP SHIFT REGISTER 430 are respectively received into sixteen resistive dividers consisting of resisters of R1-R16 (connecting to INVERTING VOLTAGE TO CURRENT CONVERTER 433) respectively connecting to resistors R20-R36 (connecting to INVERTING VOLTAGE TO CURRENT CONVERTER 434). The individual values of resistors R1-R16 equal the values of resistors R36-R20 respectively. Both sets of resisters R1-R16 and R20-R36, and the voltage dividers so formed, are chosen to produce a linear step motion of the driven motor upon each microstep. These linear step motion signals developed are approximately sine and cosine voltage signals. This is illustrated wherein the "SINE" signal, developed by stepwise increments of voltage provided from the Q1-Q16 outputs of the RECIRCULATING 16 STEP SHIFT REGISTER 430, is received at VOLTAGE TO CURRENT CONVERTER 434. Likewise, the same Q1-Q16 signal outputs are channeled through the other resistive divider leg of the 16 voltage dividers to cumulatively form stepwise signal "COSINE" which is received at INVERTING VOLTAGE TO CURRENT CONVERTER 433.

The current signal output from VOLTAGE TO CURRENT CONVERTER 433 is representative of the sine function, and is received at the first windings of X MOTOR 190, which is of a step motor type. The current output of inverting voltage to current converter 433 is likewise received at the other, orthogonal, windings of X MOTOR 190. The combination of the applied sine and cosine signals position the x step motor at a particular angular rotation. This accordingly causes movement of the X-AXIS CARRIAGE 191, and of the reference LED 160 (both shown in Figure 6) in accordance with the well known operation of an x-y plotter.

Signals developed within the current sense resistors 421, or the x,y HOME SENSORS 421, are received at the LIGHT SENSOR CONDITIONING CIRCUIT AND A/D CONVERTER 413 shown in Figure 7. When these currents are zero then the carriage has finished moving to the prescribed position and may be so recognized to have finished moving by the computer. Accordingly, no stimuli are ever applied during the sequence of testing by the perimeter 100 in accordance with the present invention save that the relative positions of such stimuli, to the target LED and the intensities thereof, are precisely known and constantly monitored by the test-administrating computer.

Further in this context of the monitoring performed by the perimeter 100 in accordance with the present invention, a typical circuit for allowing the computer to sense light conditions is shown in Figure 9. A LIGHT SENSOR 400, 423 -- which may be either used to sense background illumination or the illumination intensity at any of the normally sixteen STIMULUS LEDS 150 and the typically one FIXATION TARGET LED(s) 160 --senses the light intensity with a SILICON PHOTO SENSOR 424. The current output of the SILICON PHOTO SENSOR 424 is amplified in TRANSIMPEDANCE AMPLIFIER 425. The TRANSIMPEDANCE AMPLIFIER 425 is a current to voltage amplifier providing sufficient signal input to analog to digital converter A/D 413 (partial). The A/D 413 (partial) is part of the LIGHT SENSOR CONDITIONING CIRCUIT AND A/D CONVERTER 413 previously shown in Figure 7. The output of the A/D 413 (partial) is placed on the computer BUS which is within cable 401.

By comparing the sensed light intensities to reference intensities the computer is able to accomplish several functions. The intensities of all LEDs are able to be maintained equal, and at an appropriate level relative to room illumination, at all times. It will be recalled that the level of intensity to which the STIMULUS LEDS 150 are adjusted is inversely proportional to a height (contrast sensitivity) on the island of vision. If the STIMULUS LEDS 150 are set maximally bright, then the isopter of the island of vision which is examined is the threshold limits of the island, or its "shoreline". At lower intensity levels sensed in LIGHT SENSOR 420, 423, other contours of the island of vision may be examined to generate other isopters. It should, of course, be further understood that the individual STIMULUS LEDS 150 may be varied, and dynamically varied, in intensity in order to permit performance of the static threshold, static super-threshold, and reverse kinetic perimetry methods of visual field examination with the perimeter 100 in accordance with the present invention. The predominant methods of the present invention --modified reverse kinetic perimetry (MRKP) and modified reverse kinetic perimetry with successive approximations (MRKP-SA) -- both require variations in the illumination intensity of STIMULUS LEDS 150.

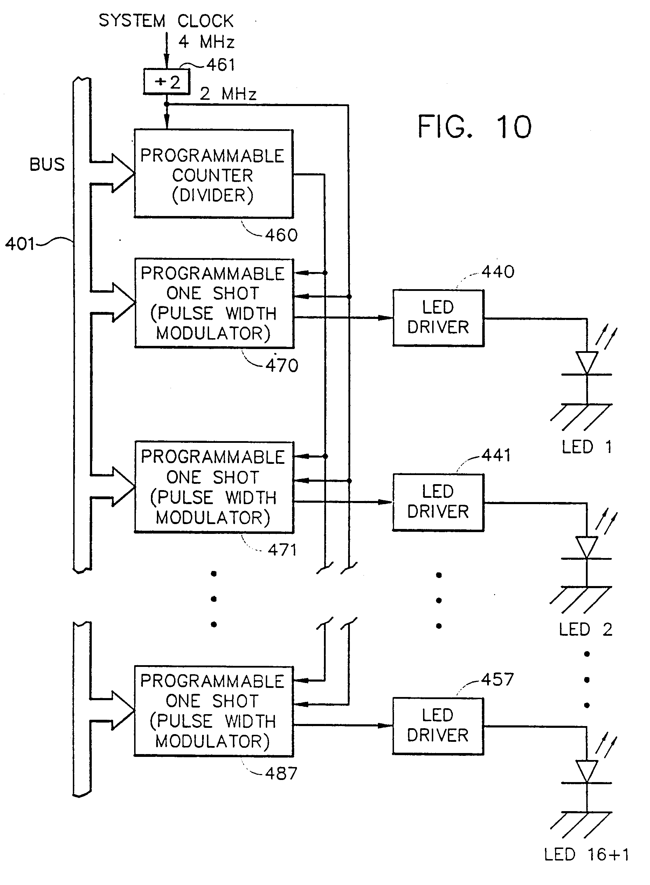

A detailed schematic diagram of FIXATION LED INTENSITY CKT 416, and the STIMULUS LED INTENSITY CKTS 419 (both previously seen in Figure 7) is shown in Figure 10. The normally sixteen STIMULUS LEDS 150 and the FIXATION TARGET LED(s) 160 are labeled LED 1 through LED 16+1. Each of the LED 1 through LED 16+1 is driven with current supplied from a corresponding LED driver 440-457. The length of time that each LED driver 440-457 is enabled to drive current through the corresponding LED 1-16+1, and the corresponding intensity of the light output from such corresponding LED, is controlled by the respective PROGRAMMABLE ONE SHOT (PULSE WIDTH MODULATOR) 470-487. Each of the PROGRAMMABLE ONE SHOT's 470-487 is programmed with the count representing a time interval during which it will produce a logically true enabling output signal via the computer bus upon cable 401.

The computer also programs, via the BUS that is within cable 401, the PROGRAMMABLE COUNTER (DIVIDER) 460. The PROGRAMMABLE COUNTER 460 receives a clock signal which is derived from the division of the SYSTEM CLOCK, nominally 4MHz, in a divide-by-two circuit 461. The resultant timing signal supplied to the PROGRAMMABLE COUNTER 460 is of 2.0 megahertz frequency. This signal is further divided within the PROGRAMMABLE COUNTER (DIVIDER) 460 in accordance with the control parameter loaded therein by the computer. Normally, the frequency is divided to a granularity wherein the counts of size which may conveniently be installed in each of the PROGRAMMABLE ONE SHOT 450-459 will permit that each of the respective LED 1 - LED 16+1 shall be maintained at some duty cycle between 0% and 100%. Normally the granularity, or single bit division, of the count is at a very refined level, typically less than 1/10,000 of the total duty cycle. The illumination intensity is correspondingly controllable with great exactitude.

Referring again to Figure 6, at the conclusion of testing (which may either prolonged or foreshortened in accordance with the computer determinations of test progress), the PERSONAL COMPUTER MOTHERBOARD 400 is able to calculate the visual field of the subject from the cumulative responses to the administered test regimen. This calculated visual field is preferably plotted in a conventional manner upon chart paper 181, which is normally 11" by 17" in size. In order to do so, the computer causes the PEN LIFT DRIVER 424 within I/O BOARD 410 to selectively enable and disable the PEN LIFT SOLENOID 183 (all shown in Figure 7), while simultaneously causing movement of X axis carriage 191 and the y AXIS CARRIAGE 192 to which the pen and PEN LIFT SOLENOID 183 is affixed, so as to cause the plotting of the isopters or other curves representative of the visual field of the eye. At the conclusion of this operation, the tray 180 containing the chart paper 181 may be slid from its position within the housing 111 to the Perimeter 100, and the chart paper 181 may be withdrawn for analysis by attending personnel and/or for display to the test subject.

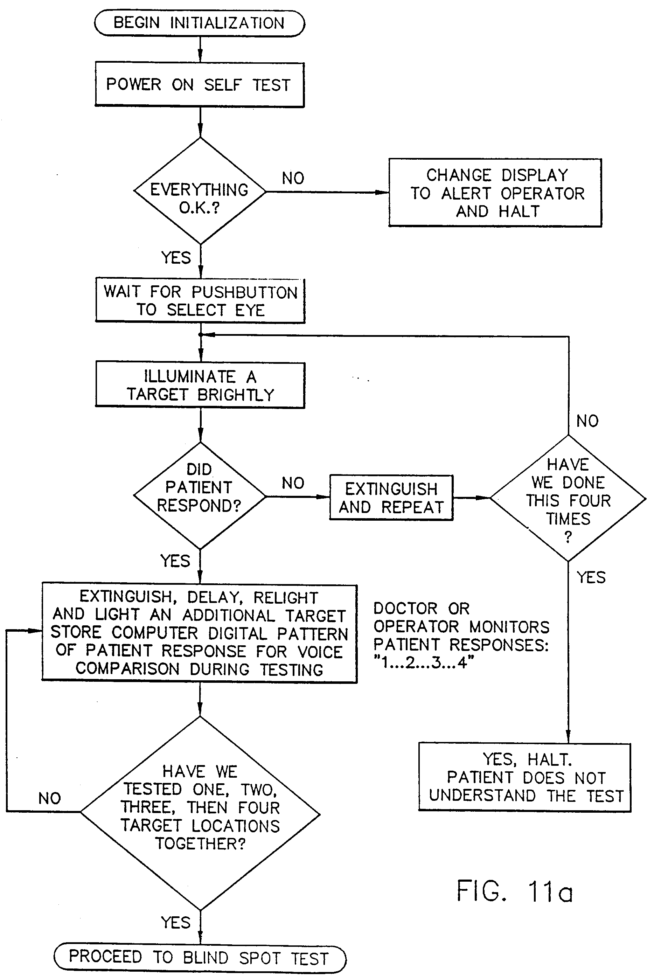

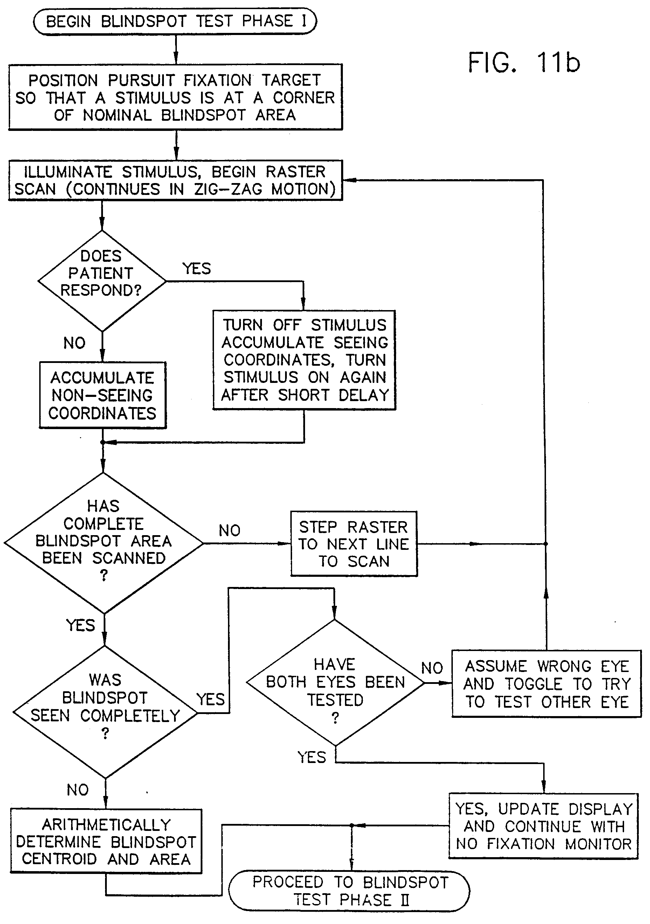

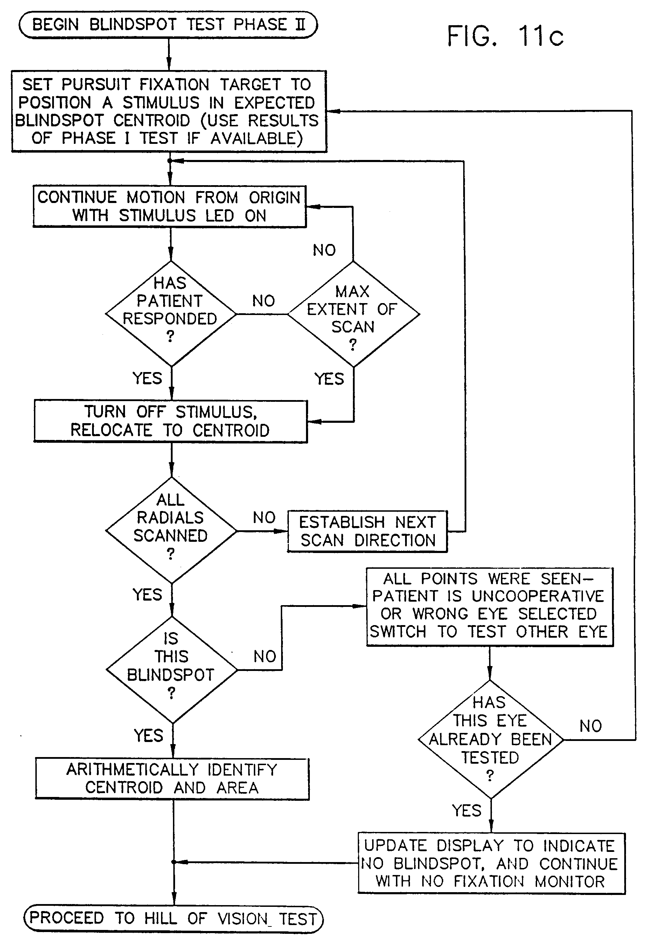

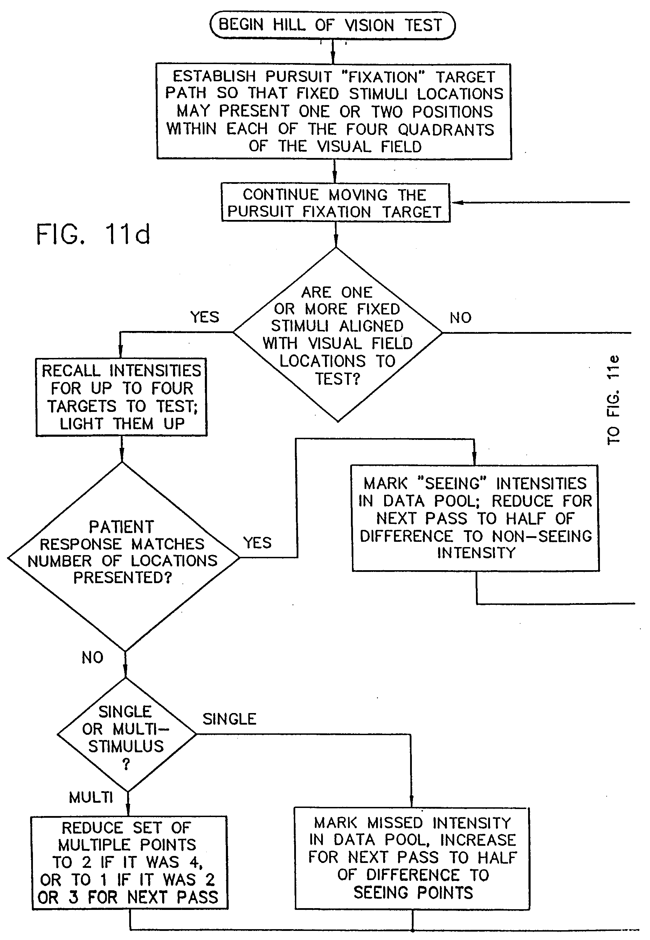

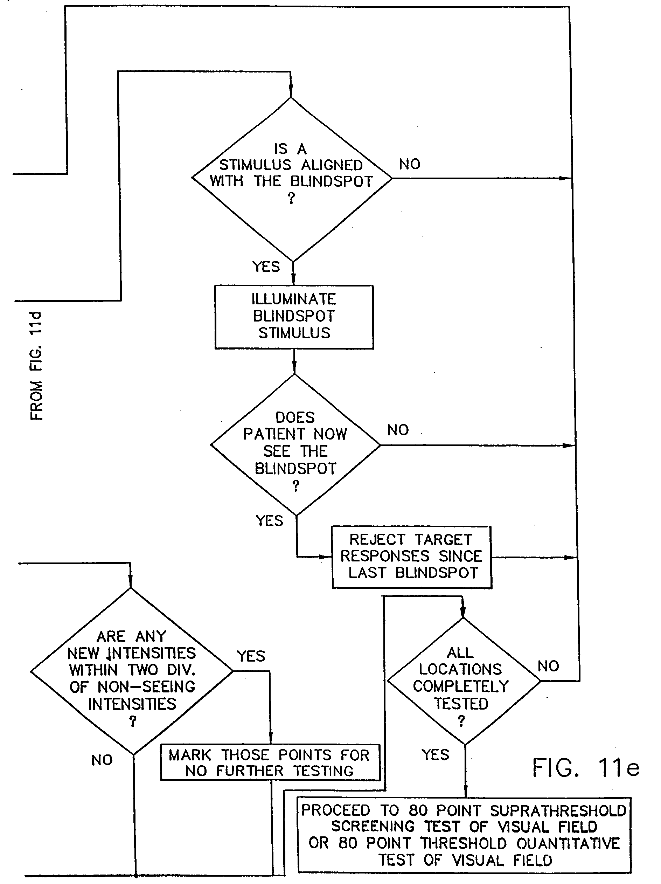

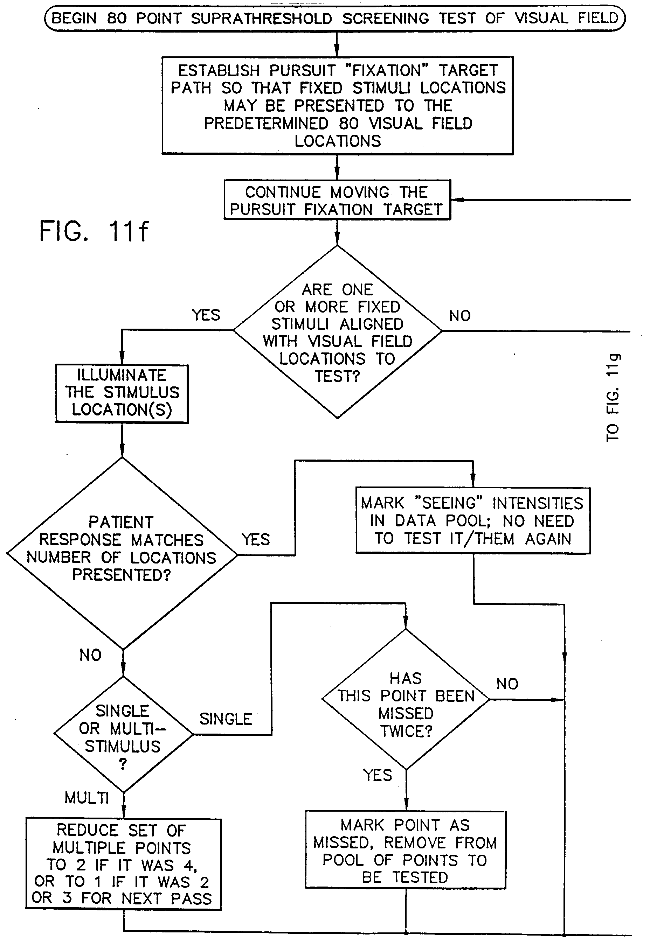

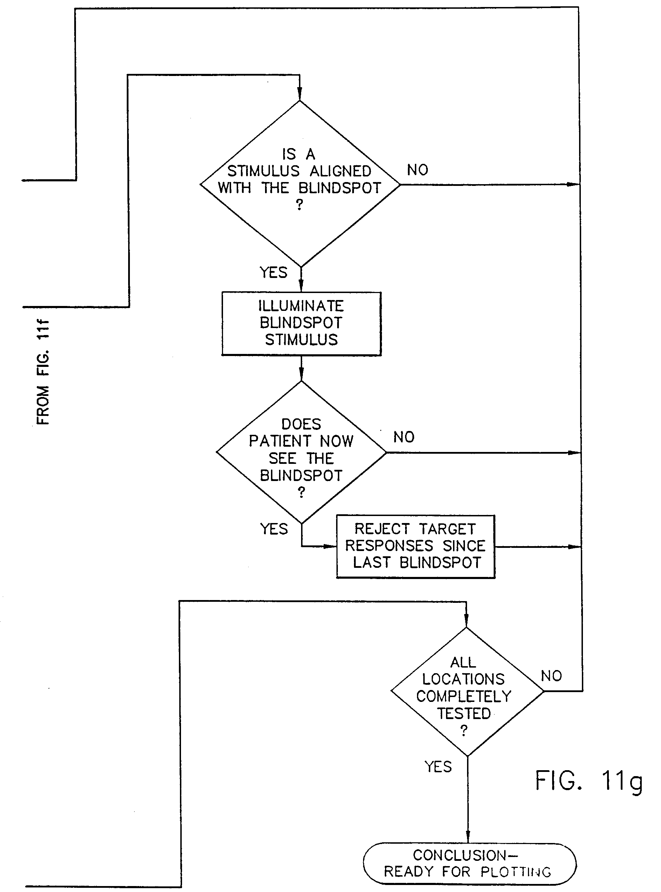

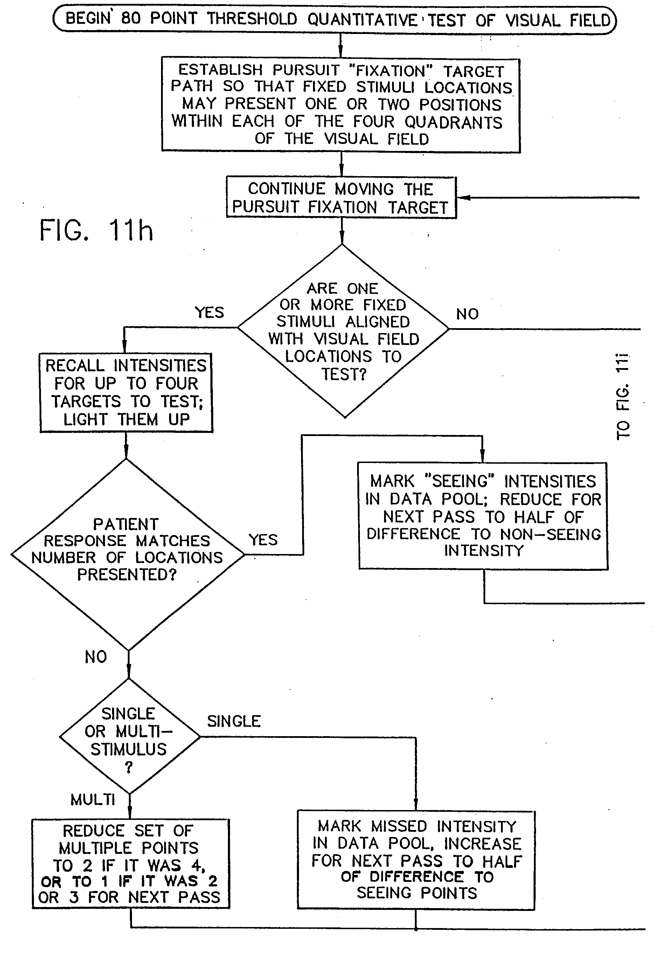

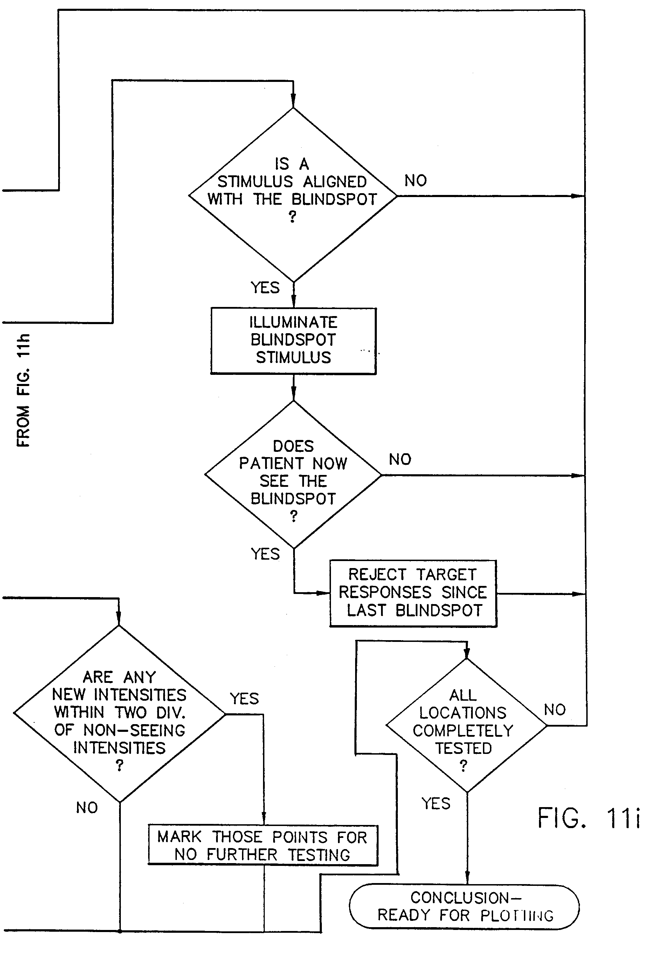

A flow chart of the preferred embodiment of the software executed by the embedded PERSONAL COMPUTER MOTHER BOARD 400 within the ocular kinetic perimetry instrument, or perimeter 100, in accordance with the present invention is shown in Figure 11, consisting of Figures 11a through 11i. The software flowchart for initialization of the perimeter, and for the training of the optional perimeter voice recognizer circuitry to the verbal response of a particular patient, is shown in Figure 11a. The voice recognizer training sequence, which is quite short, requires the participation of a doctor or other perimeter operator to determine that the patient is indeed voicing the correct response, i.e., "one", "two", "three" or "four".

The perimeter will digitalize and store any patient voiced response. The doctor or operator monitors the perimeter display, and ensures that the voiced response made to the perimeter by the patient is correctly indicative of the number of targets which are illuminated. The patient is instructed not to talk save for the voiced response, and the doctor or operator also does not talk. If the patient unduly hesitates, voices extraneous words, and/or other circumstances disrupt the training of the voice recognizer then it is a simple matter to recommence the initialization and voice recognization training sequence.

The software controlling the performance of a blind spot test phase 1 by the perimeter performing the method of reverse kinetic perimetry (RKP) is flow charted in Figure 11b. This procedure is performed in order to locate the center of the blind spot. It is more tolerant of patient blind spot alignment than the blind spot test phase 2 flow charted in Figure 11c. At the conclusion of the blind spot test phase 1 the computer of the perimeter arithmetically computes a centroid and area to the blind spot.

A flow chart of the software for performing the blind spot test phase 2 again by the method of reverse kinetic perimetry (RKP) is shown in Figure 11c. This blind spot determination is more exacting to define the extent of the blind spot than the phase 1 determination flow-charted in Figure 11b. With some knowledge of the blind spot centroid and area resultant from the performance of test phase 1, stimuli are moved radially outward from the previously determined centroid of the blind spot. The patient responses to such stimuli further define the location and extent of the blind spot with exactitude. From this data that maps the blind spot the computer again arithmetically computes both the centroid and the area of the blind spot.

There are several uses for the determined location and extent of the blind spot. By knowing where the patient's eye's blind spot is at each fixation of the patient's eye the computer intermittently presents light stimuli within the patient's blind spot during the course of testing of visual sensitivity. If the patient responds to these presentations then the eye is not properly fixating (or tracking) the (moving) fixation target.

Another use of the computed blind spot is to reveal whether such blind spot is the normally sized blind spot which is present within all human eyes, or is instead an extensive area representing a scotoma. The determination transpiring within blind spot test phases 1 and 2 enables the perimeter, during its later performance of modified reverse kinetic perimetry (MRKP), to display appropriate stimuli. For example, plural targets that are tracked by the eye focusing at a point between the targets will permit a person with a scotoma such as, for example, a scotoma extending to within the area of the fovea, to visually track a moving target.

It should be understood that to this point the computer has not, and is not, testing the extent of the field of vision. The blind spot test phases 1 and 2 are simply to determine the extent and location of the natural blind spot within the patient's field of vision, and to permit the presentation of test stimuli that appropriately compensate for the extent and location of the blind spot. In a normal eye, which exhibits good vision at the fovea, the arithmetically calculated centroid and area of the blind spot will be indicative of the actual blind spot of the normal eye. In this case, no special presentation of visual stimuli will be required during the ensuing reverse optical perimetry testing, and a single moving target will suffice to be tracked by the eye of the patient. If, however, the patient exhibits a blind spot at his/her fovea centralis then multiple targets are presented in positions surrounding the blind spot and at an adequate separation therefrom so as to be detectable by the patient during the ensuing reverse optical perimetry testing. The partially blind patient will then be instructed to "look to the center of the group of moving lights."

The blind spot test phases 1 and 2 are true (unmodified) reverse kinetic perimetry (RKP). This RKP method is not the predominant method for the actual testing of the visual field.

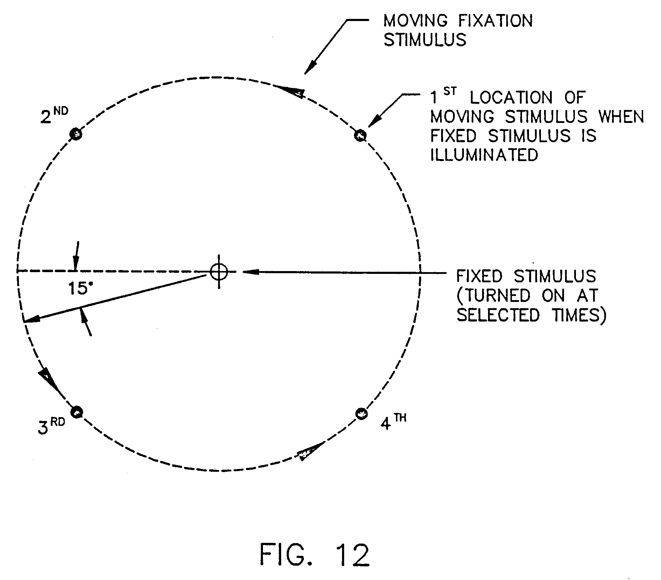

The software controlling the preliminary hill of vision test, which software controls the perimeter to perform modified reverse kinetic perimetry with successive approximations (MRKP-SA), is flow-charted in Figures 11d and 11e. The purpose of the test that is flow-charted in Figures 11d and 11e is to preliminarily determine the rough contours of the hill of vision. The computer causes the perimeter to present typically four test stimuli upon for iterations each, and to accumulate data responsively thereto. The location within the field of vision of the typically four points that are presented and tested is shown in Figure 12.

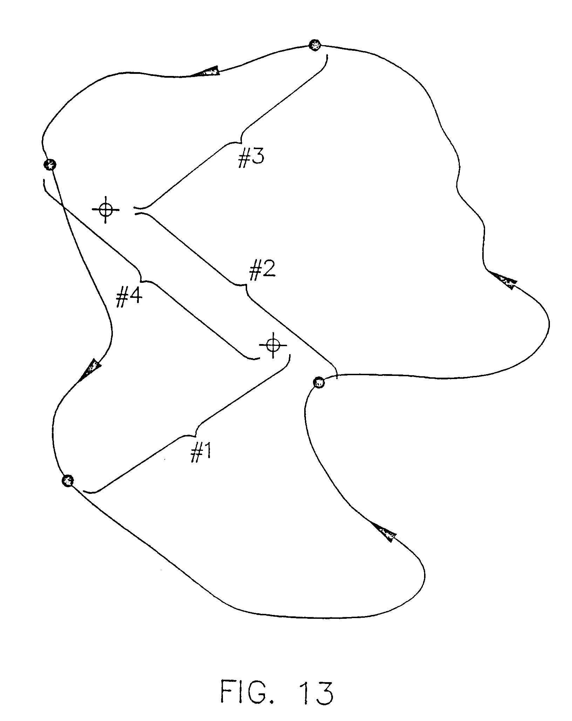

The typical manner of presentation is diagrammatically illustrated in Figure 13. The moving fixation target traces a predetermined path under computer control. Fixed position test stimuli (of which an arbitrary two only are illustrated to be used) are momentarily illuminated when the fixation target is of an appropriate angle and separation. The patient response to each single illumination is processed. Normally the first iterative illumination at each of the typically four field of vision points is suprathreshold, or at a level likely to be seen. A next successive illumination is typically subthreshold. Illuminations continue for each point of successively closer approximations driven to be of greater or lesser illumination intensity by actual patient responses. Normally three only iterations for each of the four points, or twelve total trials, suffice for a sufficiently accurate characterization of the visual sensitivity of the four points. From this actual visual sensitivity a complete "shrunken" island of vision that is customized for the individual patient is calculated by the computer.

Returning to Figures 11d and 11e, the course of sequential testing by the MRKP-SA method may be observed therein. The accumulated data actually permits the computer to calculate not one but two pseudo "hills of vision" which are respectively inside (smaller than) and outside (bigger than) the actual hill of vision. The smaller hill of vision is the assured "seen" sensitivity of the patient's eye. In other words, any test stimuli presented at the appropriate intensity and at an appropriate location so as to be within this pseudo hill of vision are assured to be seen by the patient save that the patient's eye exhibits visual defects. The larger, outside, pseudo hill of vision represents the assured "non-seen" locations and intensities that are outside the hill of vision of the patient. Any stimuli at an intensity and a displacement that is outside of this pseudo hill of vision is assuredly not detectable by the patient. The intensity of stimuli presented in the patient's blind spot is normally suprathreshold to this "outside" hill of vision.

The computerized perimeter in accordance with the present invention continues from the preliminary modified reverse kinetic perimetry with successive approximations (MRKP-SA) testing to still further testing in order to definitively quantify the actual, precise, hill of vision of a particular patient. This further testing may be either by the modified reversed kinetic perimetry (MRKP) method flow charted in Figures 11f and 11g or by the modified reverse kinetic method with successive approximations (MRKP-SA) flow charted in Figures 11h and 11i. It is preferably by the modified reverse kinetic perimetry (MRKP) method. The MRKP method preferably presents suprathreshold stimuli to the shrunken hill of vision. The software flow chart for the presentation of an 80 point suprathreshold MRKP screening test of the visual field is shown in Figures 11f and 11g. The software flow chart for the presentation of an 80 point threshold quantative MRKP-SA test of the visual field is shown in Figures 11h and 11i. Both tests produce a highly accurate three dimensional map of the contours of the actual patient hill of vision. This map is plotted for interpretation by a medical professional.

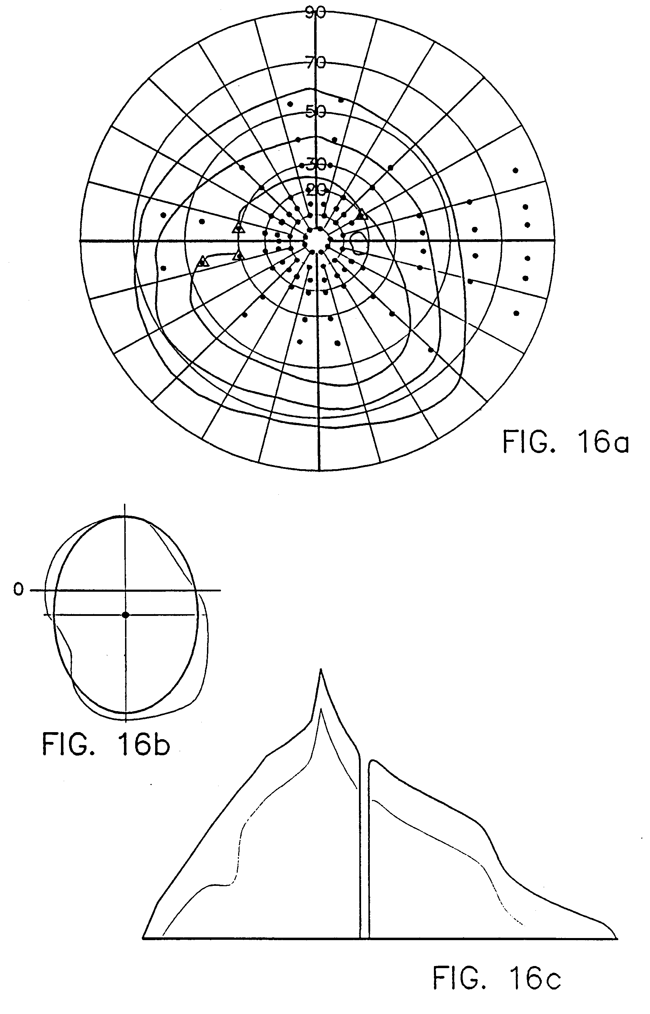

The preferred location of the eighty points tested for, by example, the left eye during each quantitative MRKP or MRKP-SA test of the visual field is diagrammed in Figure 15. The corresponding eighty points for the testing of the visual field of the right eye, over the top of which are drafted 3 isopter lines of constant visual sensitivity, are shown in Figure 16a. Defects in the visual field, or points whereat the visual sensitivity tests subthreshold to the shrunken island of vision are marked by triangles.

A graphical plot of the inner isopter of particular interest from Figure 16a versus the normal isopter of a standard sensitivity visual field calibrated to the individual patient's individual eye is shown in Figure 16b. Still another, cross-sectional, profile of the visual field, taken along horizontal axis line "0" shown in Figure 16b, is shown in Figure 16c. The top curve within Figure 16c represents a normal hill of vision for a person of the patient's age. The lower curve represents the patient's actual vision. The eye shows a defect that may be associated by a trained ophthalmologist with the probable pathological condition of glaucoma.

By the IBM PC-compatible computer mother board within the optical perimeter in accordance with the present invention, software routines control interrupt handling, motor drive control, and display drive control. It is thus specific to the particular hardware, including an x-y plotter, that is employed. Software also controls the conduct of automated perimetry. The software, divided into easily recognizable subroutines, is general to perform the indicated functions which, in aggregate, comprise the preferred methods and sequences of testing in accordance with the present invention. Modifications, or alterations, to this software will be perceived to be possible in accordance with adaptations of the present invention for performing tests other than reverse optical kinetic perimetry and its derivatives.

In accordance with the preceding discussion, it will be recognized that the present invention offers considerable flexibility, efficiency, and effectiveness in the conduct of totally automated testing of the visual field. The automated testing that transpires is nearly foolproof. The preferred embodiment of the perimeter 100 in accordance with the present invention calibrates itself. It will pause during the conduct of testing, even aborting if necessary, if no rational stimuli are received from the patient. Additionally, although a patient who is either incompetent, malingering, or obstructing the course of testing may defeat the regimen of testing by refusing to respond to stimuli, it is difficult for a patient to fake better results than are legitimately reflective of the extent and acuity of the patient's visual field. Cooperative patients generally find the test regimen easy, quick, and painless. Uncooperative test takers, or malingers, are not able to generate responses that produce test results which are subject to misinterpretation or, in most cases, to register sufficiently adequate progress during testing so that the automated perimeter will not cease testing and alert the operator. The appropriate responses to uncooperative, or malingering, test subjects are of particular importance where a machine is used to screen a large population, such as recruits to the military forces, wherein the cooperation of the individual subjects is uncertain.

In accordance with the preceding remarks, both the preferred method and the preferred embodiment of an apparatus in accordance with the present invention are obviously subject to variation. The movement of the fixation point light source could have been accomplished by selective illuminations of elements within an array of illuminatable light sources. Normally, the elements would be successively illuminated one to the next thereby causing a stepping progression in the position of the light source which is fixated by the eye of the patient. Alternatively the invention could be implemented by means of a conventional target light source-directed via motors, lenses and mirrors to a planar or curved screen which contains a multiplicity of fixed light sources as stimuli.

The presentation field of the perimeter need not have been a flat tangent screen. The methods of the present invention could be implemented within a hemispherical bowl perimeter. The number of stimulus light sources need not have been set at sixteen, need not have been light emitting diodes, and need not have been fixed in position. The computer need not have used the same mechanism which is otherwise used to move the light sources during the course of testing to generate, in graphical chart form, the test results but could instead be connected, as by a data bus, to a conventional graphics printer or the like.

Corresponding to these and other obvious modifications, the present invention should be perceived broadly. In particular, the scope of the invention should be determined by the following claims, only, and not solely in accordance with that particular embodiment within which the invention has been taught.

## Claims

1. A method of kinetic perimetry for examining the visual sensitivity of a patient's eye, comprising the steps of
illuminating a first reference light source (160) that is fixated by the patient's eye;
illuminating a second stimulus light source (150) while it assumes various distances of separation from, and angles relative to, the first reference light source (160);
indicating those ones of the second stimulus light sources (150) that are visually detected by the patient's eye while it is fixated to the first reference light source (160); and
recording the various angles and distances of separation between the first reference light source (160) and the detected second stimulus light sources (150) as an indication of the visual field of the patient's eye;
the method being **characterized** in that
said first reference light source (160) is moved while it is fixated substantially continuously by the patient's eye, so that the patient's eye must move in order to follow the movement of the first reference light source (160); whereas
said second stimulus light source (150) is held fixed while the first reference light source (160) is moved, so that the second stimulus light source (150) is detected by the patient in the course of the movement of the patient's eye as it follows the first reference light source (160).

2. The method as in claim 1, wherein a plurality of second stimulus light sources (150) are illuminated simultaneously.

3. The method as in claim 1 or 2, wherein said at least one second stimulus light source (150) is illuminated momentarily at times, the at least one second stimulus light source (150) being at predetermined distances of separation from, and angles relative to, the first reference light source (160) at the times of its momentary illuminations.

4. The method as in claim 3, wherein the illumination intensity of the at least one second stimulus light source (150) upon the times of its momentary illuminations is varied to be in successive approximations both suprathreshold and subthreshold to the visual sensitivity of the patient's eye.

5. The method as in claim 2, wherein the response of the patient with regard to the number of detected stimulus light sources (150) is interpreted in a voice recognition system (418) to produce a digital quantity representative of the particular number spoken.

6. A perimeter apparatus comprising in collective operation:
means for activating a first reference light source (160) that is fixated by the patient's eye;
means (190-195) for moving said first reference light source (160) while it is fixated substantially continuously by the patient's eye;
means for activating at least one second stimulus light source (150) while it assumes various distances of separation from, and angles relative to, the first reference light source (160);
means for indicating (170) in dependence on various angles and distances of separation between the first reference light source (160) and the second stimulus light sources (150) those ones of the second stimulus light sources (150) that are visually detected by the patient's eye while it is fixated to the first reference light source (160);
means for recording (180) the various angles and distances of separation between the first reference light source (160) and the detected second stimulus light sources (150) as an indication of the visual field of the patient's eye; and
means (400, 410) for controlling the operation of the above mentioned means (150; 160; 170; 180; 190-195) in accordance with the method of kinetic perimetry as defined in any of the claims 1 to 5.

7. The apparatus of claim 6, comprising further a computer (400) automating the moving of the first reference light source (160) by the means (192, 193) for moving.

8. The apparatus of claim 6, comprising further a plurality of second stimulus light sources (150); and a frame (110) positionally fixing the plurality of second stimulus light sources.

9. The apparatus of claim 6, comprising further an x-y plotter (192, 193) for moving said first reference light source (160).

## Patentansprüche

1. Kinetisches Perimetrieverfahren zum Überprüfen des Sehvermögens des Auges eines Patienten, mit den Schritten
des Aufleuchtenlassens einer ersten Bezugs-Lichtquelle (160), die vom Auge des Patienten fixiert wird;
des Aufleuchtenlassens einer zweiten Stimulations-Lichtquelle (150), während sie verschiedene Abstände und Winkel relativ zur ersten Bezugs-Lichtquelle (160) aufweist;
des Anzeigens derjenigen der zweiten Stimulations-Lichtquellen (150), die visuell vom Auge des Patienten erfaßt werden, während es auf die erste Bezugs-Lichtquelle (160) fixiert ist; und
des Aufzeichnens der verschiedenen Winkel und Abstände zwischen der ersten Bezugs-Lichtquelle (160) und den erfaßten zweiten Stimulations-Lichtquellen (150) als Anzeige des Sehfeldes des Auges des Patienten;
dadurch **gekennzeichnet**, daß
die erste Bezugs-Lichtquelle (160) bewegt wird, während sie im wesentlichen ununterbrochen vom Auge des Patienten fixiert wird, so daß sich das Auge des Patienten bewegen muß, um der Bewegung der ersten Bezugs-Lichtquelle (160) zu folgen; während
die zweite Stimulations-Lichtquelle (150) festgehalten wird, während sich die erste Bezugs-Lichtquelle (160) bewegt, so daß die zweite Stimulations-Lichtquelle (150) vom Patienten im Laufe der Bewegung des Auges des Patienten, wenn es der ersten Bezugs-Lichtquelle (160) folgt, erfaßt wird.

2. Verfahren nach Anspruch 1, wobei eine Anzahl von zweiten Stimulations-Lichtquellen (150) gleichzeitig beleuchtet wird.

3. Verfahren nach Anspruch 1 oder 2, wobei vorübergehend wenigstens eine zweite Stimulations-Lichtquelle (150) beleuchtet wird, wobei sich die wenigstens eine zweite Stimulations-Lichtquelle (150) zu den Zeitpunkten der vorübergehenden Beleuchtung in vorgegebenen Abständen von und in vorgegebenen Winkeln zu der ersten Bezugs-Lichtquelle (160) befindet.

4. Verfahren nach Anspruch 3, wobei die Leuchtintensität der wenigstens einen zweiten Stimulations-Lichtquelle (150) zu den Zeitpunkten der vorübergehenden Beleuchtung verändert wird, um in aufeinanderfolgenden Annäherungen sowohl unter als auch über der Schwelle des Sehvermögens des Auges des Patienten zu liegen.

5. Verfahren nach Anspruch 2, wobei die Reaktion des Patienten hinsichtlich der Anzahl von erfaßten Stimulations-Lichtquellen (150) in einem Spracherkennungssystem (418) interpretiert wird, um eine digitale Größe zu erzeugen, die die jeweils ausgesprochene Zahl darstellt.

6. Perimetervorrichtung, die zusammenwirkend enthält
eine Einrichtung zum Aktivieren einer ersten Bezugs-Lichtquelle (160), die vom Auge eines Patienten fixiert wird;
eine Einrichtung (190-195) zum Bewegen der ersten Bezugs-Lichtquelle (160), während sie vom Auge des Patienten im wesentlichen ununterbrochen fixiert wird;
eine Einrichtung zum Aktivieren wenigstens einer zweiten Stimulations-Lichtquelle (150), während sie verschiedene Abstände und Winkel relativ zur ersten Bezugs-Lichtquelle (160) einnimmt;
eine Einrichtung (170) zum Anzeigen derjenigen der zweiten Stimulations-Lichtquellen (150), die visuell vom Auge des Patienten erfaßt werden, während es auf die erste Bezugs-Lichtquelle (160) fixiert ist, in Abhängigkeit von den verschiedenen Winkeln und Abständen zwischen der ersten Bezugs-Lichtquelle (160) und den zweiten Stimulations-Lichtquellen (150);
eine Einrichtung (180) zum Aufzeichnen der verschiedenen Winkel und Abstände zwischen der ersten Bezugs-Lichtquelle (160) und den erfaßten zweiten Stimulations-Lichtquellen (150) als Anzeige des Sehfeldes des Auges des Patienten; und
eine Einrichtung (400, 410) zum Steuern des Betriebs der genannten Einrichtungen (150; 160; 170; 180; 190-195) gemäß dem kinetischen Perimetrieverfahren nach einem der Ansprüche 1 bis 5.

7. Vorrichtung nach Anspruch 6, mit einem Computer (400), der die Bewegung der ersten Bezugs-Lichtquelle (160) mittels der Bewegungseinrichtung (192, 193) automatisiert.

8. Vorrichtung nach Anspruch 6, mit einer Anzahl von zweiten Stimulations-Lichtquellen (150); und mit einem Rahmen (110), der die Positionen der Anzahl von zweiten Stimulations-Lichtquellen (150) fixiert.

9. Vorrichtung nach Anspruch 6, mit einem X-Y-Plotter (192, 193) zum Bewegen der ersten Bezugs-Lichtquelle (160).

## Revendications

1. Méthode de périmétrie cinétique pour examiner la sensibilité visuelle de l'oeil d'un patient, comprenant les étapes consistant à :
effectuer un éclairage par une première source de lumière de référence (160) qui est fixée par l'oeil du patient ;
effectuer un éclairage par une deuxième source de lumière de stimulation (150), alors qu'elle adopte diverses distances de séparation depuis la première source de lumière de référence (160) et divers angles par rapport à celle-ci ;
indiquer lesquelles, parmi les deuxièmes sources de lumière de stimulation (150), sont visuellement détectées par l'oeil du patient tandis qu'il fixe la première source de lumière de référence (160) ; et
enregistrer les divers angles et distances de séparation entre la première source de lumière de référence (160) et les deuxièmes sources de lumière de stimulation détectées (150) comme étant une indication du champ visuel de l'oeil du patient ;
la méthode étant caractérisée en ce que :
ladite première source de lumière de référence (160) est déplacée tandis qu'elle est fixée pratiquement en continu par l'oeil du patient, de telle sorte que l'oeil du patient doive se déplacer afin de suivre le mouvement de la première source de lumière de référence (160) ; tandis que
ladite deuxième source de lumière de stimulation (150) est maintenue fixe alors que la première source de lumière de référence (160) est déplacée, de telle sorte que la deuxième source de lumière de stimulation (150) soit détectée par le patient au cours du déplacement de l'oeil du patient pendant qu'il suit la première source de lumière de référence (160).

2. Méthode selon la revendication 1, dans laquelle une pluralité de deuxièmes sources de lumière de référence (150) sont allumées en même temps.

3. Méthode selon la revendication 1 ou 2, dans laquelle ladite deuxième source de lumière de stimulation (150) au nombre d'au moins une est allumée momentanément à certains moments, la deuxième source de lumière de stimulation (150) au nombre d'au moins une étant située à des distances de séparation prédéterminées depuis la première source de lumière de référence (160) et à des angles prédéterminés par rapport à celle-ci, au moment de ses illuminations momentanées.

4. Méthode selon la revendication 3, dans laquelle l'intensité d'éclairage de la deuxième source de lumière de stimulation (150) au nombre d'au moins une au moment de ses illuminations momentanées varie de façon à réaliser des approximations successives du seuil maximal et du seuil minimal de la sensibilité visuelle de l'oeil du patient.

5. Méthode selon la revendication 2, dans laquelle la réponse du patient, en ce qui concerne le nombre de sources de lumière de stimulation (150) détectées est interprétée dans un système de reconnaissance vocale (418) pour produire une quantité numérique représentative du nombre particulier annoncé.

6. Appareil périmétrique comprenant, en fonctionnement conjoint :
des moyens pour activer une première source de lumière de référence (160) qui est fixée par l'oeil du patient ;
des moyens (190-195) pour déplacer ladite première source de lumière de référence (160) tandis qu'elle est fixée pratiquement en continu par l'oeil du patient ;
des moyens pour activer au moins une deuxième source de lumière de stimulation (150) alors qu'elle adopte diverses distances de séparation depuis la première source de lumière de référence (160) et divers angles par rapport à celle-ci ;
des moyens pour indiquer (170), en fonction des divers angles et distances de séparation entre la première source de lumière de référence (160) et les deuxièmes sources de lumière de stimulation (150), lesquelles, parmi les deuxièmes sources de lumière de stimulation (150), sont visuellement détectées par l'oeil du patient alors qu'il fixe la première source de lumière de référence (160) ;
des moyens pour enregistrer (180) les divers angles et distances de séparation entre la première source de lumière de référence (160) et les deuxièmes sources de lumière de stimulation détectées (150) comme étant une indication du champ visuel de l'oeil du patient ; et
des moyens (400, 410) pour commander le fonctionnement des moyens mentionnés ce-dessus (150 ; 160 ; 170 ; 180 ; 190 - 195) conformément à la méthode de périmétrie cinétique telle que définie dans l'une quelconque des revendications 1 à 5.

7. Appareil selon la revendication 6, comprenant en outre un ordinateur (400) automatisant le déplacement de la première source de lumière de référence (160) grâce aux moyens (192, 193) de déplacement.

8. Appareil selon la revendication 6, comprenant en outre une pluralité de deuxièmes sources de lumière de stimulation (150) ; et un bâti (110) fixant en position la pluralité de deuxièmes sources de lumière de stimulation.

9. Appareil selon la revendication 6, comprenant en outre un traceur x-y (192, 193) pour déplacer ladite première source de lumière de référence (160).
